(19) Europäisches Patentamt
European Patent Office
Office européen des brevets

(11) Veröffentlichungsnummer: **0 022 183**
B1

(12) **EUROPÄISCHE PATENTSCHRIFT**

(45) Veröffentlichungstag der Patentschrift: **23.06.82**

(21) Anmeldenummer: **80103232.7**

(22) Anmeldetag: **11.06.80**

(51) Int. Cl.³: **C 07 C 13/28**, C 07 C 21/24, C 07 C 43/21, C 07 C 43/225, C 07 C 69/63, C 07 C 69/035, C 07 C 1/22, C 07 C 17/32, C 07 C 41/00, C 09 K 3/34, G 02 F 1/13

(54) **Cyclohexylbiphenyle, Verfahren zu ihrer Herstellung, diese enthaltende Dielektrika und elektrooptisches Anzeigeelement.**

(30) Priorität: **06.07.79 DE 2927277**

(43) Veröffentlichungstag der Anmeldung: **14.01.81 Patentblatt 81/2**

(45) Bekanntmachung des Hinweises auf die Patenterteilung: **23.06.82 Patentblatt 82/25**

(84) Benannte Vertragsstaaten: **AT CH DE FR GB IT LI NL**

(56) Entgegenhaltungen:
**DE-A-2 636 684**
**DE-A-2 701 591**
**DE-A-2 802 588**
**Patents Abstracts of Japan Band 3, Nr. 100, 24. August 1979 Seite 70C56**

(73) Patentinhaber: **Merck Patent Gesellschaft mit beschränkter Haftung, Frankfurter Strasse 250, D-6100 Darmstadt (DE)**

(72) Erfinder: **Eidenschink, Rudolf, Dr., Dessauer Strasse 57, D-6110 Dieburg (DE)**
Erfinder: **Erdmann, Dietrich, Dr., Heideweg 4, D-6109 Mühltal, (DE)**
Erfinder: **Krause, Joachim, Dr., Samuel-Morse-Weg 10, D-6110 Dieburg (DE)**
Erfinder: **Pohl, Ludwig, Dr., Hoffmannstrasse 54, D-6100 Darmstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

EP 0 022 183 B1

Cyclohexylbiphenyle, Verfahren zu ihrer Herstellung, diese enthaltende Dielektrika und elektrooptisches Anzeigeelement

Für elektrooptische Anzeigeelemente werden in zunehmendem Maße die Eigenschaften nematischer oder nematisch-cholesterischer flüssigkristalliner Materialien ausgenutzt, ihre optischen Eigenschaften wie Lichtabsorption, Lichtstreuung, Doppelbrechung, Reflexionsvermögen oder Farbe unter dem Einfluß elektrischer Felder signifikant zu verändern. Die Funktion derartiger Anzeigeelemente beruht dabei beispielsweise auf den Phänomenen der dynamischen Streuung, der Deformation aufgerichteter Phasen, dem Schadt-Helfrich-Effekt in der verdrillten Zelle oder dem cholesterisch-nematischen Phasenübergang.

Für die technische Anwendung dieser Effekte in elektronischen Bauelementen werden flüssigkristalline Dielektrika benötigt, die einer Vielzahl von Anforderungen genügen müssen. Besonders wichtig sind hier die chemische Beständigkeit gegenüber Feuchtigkeit, Luft und physikalischen Einflüssen wie Wärme, Strahlung im infraroten, sichtbaren und ultravioletten Bereich und elektrische Gleich- und Wechselfelder. Ferner wird von technisch verwendbaren flüssigkristallinen Dielektrika eine flüssigkristalline Mesophase im Temperaturbereich von mindestens +10°C bis +50°C, bevorzugt von 0°C bis 60°C, und eine möglichst niedrige Viskosität bei Raumtemperatur, die vorzugsweise nicht mehr als $70 \cdot 10^{-3}$ Pa · s betragen soll, gefordert. Schließlich dürfen sie im Bereich des sichtbaren Lichtes keine Eigenabsorption aufweisen, d. h. sie müssen farblos sein.

Es ist bereits eine Anzahl von flüssigkristallinen Verbindungen bekannt, die den an Dielektrika für elektronische Bauelemente gestellten Stabilitätsanforderungen genügen und auch farblos sind. Hierzu gehören insbesondere die in der DE-OS 2 139 628 beschriebenen p,p'-disubstituierten Benzoesäurephenylester und die in der DE-OS 2 636 684 beschriebenen p,p'-disubstituierten Phenylcyclohexanderivate. In beiden genannten Verbindungsklassen wie auch in anderen bekannten Reihen von Verbindungen mit flüssigkristalliner Mesophase gibt es keine Einzelverbindungen, die in dem geforderten Temperaturbereich von 10°C bis 60°C eine flüssigkristalline nematische Mesophase ausbilden. Es werden daher in der Regel Mischungen von zwei oder mehreren Verbindungen hergestellt, um als flüssigkristalline Dielektrika verwendbare Substanzen zu erhalten. Hierzu mischt man gewöhnlich mindestens eine Verbindung mit niedrigem Schmelz- und Klärpunkt mit einer anderen mit deutlich höherem Schmelz- und Klärpunkt.

Hierbei wird normalerweise ein Gemisch erhalten, dessen Schmelzpunkt unter dem der niedriger schmelzenden Komponente liegt, während der Klärpunkt zwischen den Klärpunkten der Komponenten liegt. Optimale Dielektrika lassen sich jedoch auf diese Weise nicht herstellen, da die Komponenten mit den hohen Schmelz- und Klärpunkten den Gemischen fast immer auch eine hohe Viskosität verleihen. Dadurch werden die Schaltzeiten der damit hergestellten elektro-optischen Anzeigeelemente in unerwünschter Weise verlängert.

Der Erfindung liegt die Aufgabe zugrunde, flüssigkristalline Dielektrika herzustellen, die eine nematische Phase im geforderten Temperaturbereich aufweisen und in Flüssigkristallzellen bei Raumtemperatur ausreichend kurze Schaltzeiten ermöglichen.

Aus der DE-OS 2 701 591 sind bereits Hexahydroterphenylderivate bekannt, mit denen diese Aufgabe für den Teilbereich der flüssigkristallinen Dielektrika mit positiver dielektrischer Anisotropie gelöst werden kann. Zur Herstellung flüssigkristalliner Dielektrika mit negativer dielektrischer Anisotropie, die z. B. für nach dem Prinzip der dynamischen Streuung arbeitende Flüssigkristall-Anzeigeelemente benötigt werden, sind diese Hexahydroterphenylderivate jedoch nicht brauchbar.

Es wurde nun gefunden, daß die Cyclohexylbiphenyle der Formel (I)

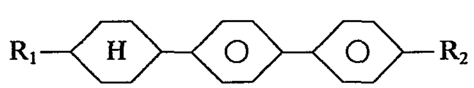

(I)

worin $R_1$ eine Alkylgruppe mit 1–12 C-Atomen und $R_2$ eine gegebenenfalls perfluorierte Alkyl-, Alkoxy- oder Alkanoyloxygruppe mit 1–12 C-Atomen bedeutet, wobei jedoch höchstens einer der Substituenten $R_1$ und $R_2$ eine verzweigte Kohlenstoffkette enthält, vorzüglich als Mischungs-Komponenten flüssigkristalliner Dielektrika geeignet sind. Die Verbindungen der Formel (I) besitzen überraschenderweise selbst flüssigkristalline Mesophasen in einem breiten Temperaturbereich, wobei die Klärpunkte in der Regel bei etwa 150°C oder darüber liegen. Derartig breite Mesophasen waren auch im Hinblick auf die strukturverwandten cyanosubstituierten Hexahydroterphenylderivate nach der DE-OS 2 701 591 nicht zu erwarten, wie beispielsweise der Vergleich zwischen den flüssigkristallinen Cyanobiphenylderivaten gemäß der DE-OS 2 356 085 und den vorbekannten nicht flüssigkristallinen p,p'-Dialkyl- bzw. Alkyl-Alkoxy-Biphenylen zeigt. Die dielektrische Anisotropie der Cyclohexylbiphenyle der Formel (I) liegt etwa zwischen −1 und +3, in der Regel um Null. Durch den Zusatz dieser Verbindungen wird der Temperaturbereich der Mesophase flüssigkristalliner Basismaterialien deutlich erweitert; in vielen Fällen wird überraschenderweise auch die Viskosität von an sich relativ niederviskosen Flüssigkristallmaterialien noch vermindert.

Gegenstand der Erfindung sind somit die Cyclohexylbiphenyle der Formel (I), Verfahren zu ihrer

Herstellung und ihre Verwendung als Komponenten flüssigkristalliner Dielektrika. Gegenstand der Erfindung sind ferner flüssigkristalline Dielektrika aus 2 oder mehreren Komponenten mit einem Gehalt an mindestens einem Cyclohexylbiphenylderivat der Formel (I) sowie elektrooptische Anzeigeelemente auf der Basis einer Flüssigkristallzelle, die ein derartiges flüssigkristallines Dielektrikum enthalten.

Der Substituent $R_1$ in den Verbindungen der Formel (I) kann geradkettig oder verzweigt sein. Wenn R geradkettig ist, also Methyl, Äthyl, n-Propyl, n-Butyl, n-Pentyl, n-Hexyl, n-Heptyl, n-Octyl, n-Nonyl, n-Decyl, n-Undecyl oder n-Dodecyl bedeutet, besitzen die dadurch charakterisierten Verbindungen der Formel (I) in der Regel besonders hohe Klärpunkte. Besonders bevorzugt sind die Verbindungen der allgemeinen Formel (I), in denen $R_1$ ein Alkylrest mit 1–10, insbesondere mit 1–8 C-Atomen ist. Gelegentlich sind jedoch auch Verbindungen der allgemeinen Formel (I) mit verzweigtem Substituenten $R_1$ von Bedeutung, da diese häufig bessere Löslichkeitseigenschaften in den üblichen flüssigkristallinen Grundmischungen aufweisen. Ferner können die Verbindungen mit einem verzweigten Substituenten $R_1$ in optisch aktiver Form hergestellt werden; diese Substanzen haben als chirale Dotierstoffe Bedeutung. Solche nicht geradkettigen Substituenten $R_1$ enthalten nicht mehr als eine Kettenverzweigung. Bevorzugte verzweigte Substituenten sind die, in denen sich an einer längeren Kohlenstoffkette in 2- oder 3-Stellung eine Methyl- oder Äthylgruppe befindet, beispielsweise 2-Methylpropyl, 2-Methylbutyl, 3-Methylbutyl, 2-Methylpentyl oder 2-Äthylhexyl.

Diese Auswahlkriterien gelten analog auch für den Substituenten $R_2$ in den Verbindungen der Formel (I), wenn dieser eine Alkylgruppe ist. Wenn $R_2$ eine Alkoxy- oder Alkanoyloxygruppe bedeutet, gelten diese Kriterien auch für den Alkylteil dieser Gruppen; zusätzlich sind darin aber auch noch in 1-Stellung verzweigte Alkylteile interessant, so daß als Alkoxygruppen $R_2$ außer den von den vorstehend genannten Alkylgruppen abgeleiteten auch noch beispielsweise 1-Methylpropyloxy, 1-Methylbutyloxy, 1-Methylpentyloxy, 1-Methylhexyloxy oder 1-Methylheptyloxy von Bedeutung sind; als verzweigte Alkanoyloxygruppen sind Isobutyryloxy und Isovaleryloxy von besonderem Interesse.

In den erfindungsgemäßen Verbindungen enthält höchstens einer der Substituenten $R_1$ und $R_2$ eine verzweigte Kohlenstoffkette.

Wenn $R_2$ eine perfluorierte Alkyl-, Alkoxy- oder Alkanoyloxygruppe ist, sind unter diesen die nicht mehr als 8 C-Atome enthaltenden, und insbesondere die mit 1–5 C-Atomen bevorzugt. Auch diese enthalten maximal nur eine Kettenverzweigung, wobei eine Trifluormethylgruppe in 2- oder 3-Stellung bevorzugt ist.

In allen erfindungsgemäßen Verbindungen sind die Substituenten am Cyclohexanring trans-ständig angeordnet; in den Formelbildern ist dies durch die schwarze Markierung an der rechten Seite des Cyclohexanringes zum Ausdruck gebracht.

Die erfindungsgemäßen Verbindungen werden in für derartige Substanzen üblicher Weise hergestellt. So werden die Verbindungen der Formel (I), worin $R_2$ eine Alkylgruppe bedeutet, erhalten, indem in einer Verbindung der Formel (II)

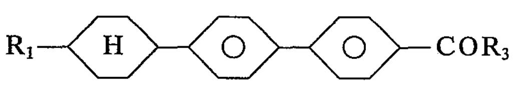

(II)

worin $R_3$ H oder Alkyl mit 1–11 C-Atomen bedeutet, die Carbonylgruppe zur Methylengruppe reduziert wird. Diese Reduktion erfolgt in an sich bekannter Weise, zum Beispiel durch katalytische Hydrierung oder mit Hydrazinhydrat und einem Alkalimetallhydroxid oder -alkoholat in einem hochsiedenden Lösungsmittel wie Diäthylenglykol oder Dimethylsulfoxid, mit Zink und Chlorwasserstoff in Diäthyläther oder mit amalgamiertem Zink in wäßriger Salzsäure. Nach an sich üblicher Aufarbeitung werden die so erhaltenen Verbindungen der Formel (I) durch fraktionierte Destillation unter vermindertem Druck oder durch Umkristallisieren aus einem geeigneten Lösungsmittel, zum Beispiel Äthanol oder Essigsäureäthylester, gereinigt.

Die Verbindungen der Formel (I), in denen $R_2$ eine Alkoxygruppe bedeutet, werden hergestellt, indem ein Keton der Formel (II), worin $R_3$ vorzugsweise Methyl bedeutet, durch Behandlung mit einem Oxidationsmittel und anschließende Hydrolyse in ein Phenol der Formel (III)

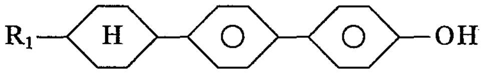

(III)

überführt wird, und dieses dann mit einem 0-alkylierenden Mittel umgesetzt wird. Als Oxidationsmittel für die Überführung des Ketons (II) in das Phenol (III) wird beispielsweise Perameisensäure oder eine andere Persäure verwendet. Die Umsetzung mit einem O-alkylierenden Mittel, zum Beispiel einem Alkyljodid oder Alkylbromid erfolgt in der Regel in Gegenwart einer Base wie Natriumhydroxid oder Natriumcarbonat in einem polaren Lösungsmittel, zum Beispiel Aceton.

Die Verbindungen der Formel (I), in denen $R_2$ eine Perfluoralkylgruppe bedeutet, werden hergestellt, indem ein Keton der Formel (IIa)

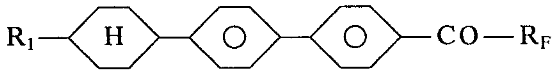 (IIa)

worin $R_F$ einen Perfluoralkylrest mit 1–11 C-Atomen bedeutet, mit Schwefeltetrafluorid umgesetzt wird. Die Ketone (IIa) werden dabei durch Umsetzung des entsprechenden 4-(4-Alkylcyclohexyl)-biphenyls mit einem Perfluorcarbonsäurehalogenid in Gegenwart eines Friedel-Crafts-Katalysators erhalten.

Die Verbindungen der Formel (I), in denen $R_2$ eine Perfluoralkoxygruppe ist, werden hergestellt, indem ein Phenol (III) mit einer Perfluorcarbonsäure unter an sich üblichen Bedingungen verestert wird und der erhaltene Ester mit Schwefeltetrafluorid in Gegenwart einer fluorhaltigen Lewis-Säure, zum Beispiel Fluorwasserstoff, Bortrifluorid oder Titantetrafluorid umgesetzt wird. Die Verbindungen der Formel (I), in denen $R_2$ eine gegebenenfalls perfluorierte Alkanoyloxygruppe ist, werden hergestellt, indem ein Phenol (III) mit einer Carbonsäure bzw. Perfluorcarbonsäure oder einem reaktiven Derivat einer solchen Säure, zum Beispiel einem Säurehalogenid, vorzugsweise Säurechlorid oder einem Säureanhydrid, zweckmäßig in Gegenwart einer Base wie zum Beispiel Pyridin oder Triäthylamin umgesetzt wird.

Die Cyclohexylbiphenyle der Formel (I) werden als Komponenten flüssigkristalliner Dielektrika zu dem Zweck verwendet, den Temperaturbereich der flüssigkristallinen Mesophase zu erweitern, insbesondere den Klärpunkt zu erhöhen. Gegenüber den bisher für diesen Zweck verwendeten Benzoyloxybenzoesäurephenylesterderivaten nach der DE-OS 2 139 628 oder den Biphenylcarbonsäurephenylesterderivaten bzw. Benzoesäurebiphenylylesterderivaten nach der DE-OS 2 450 088 bieten die erfindungsgemäßen Verbindungen den wesentlichen Vorteil, daß sie die Viskosität der flüssigkristallinen Basisdielektrika weniger erhöhen bzw. sogar herabsetzen. Aufgrund der niedrigen Werte ihrer dielektrischen Anisotropie werden sie bevorzugt als Komponenten flüssigkristalliner Dielektrika mit negativer dielektrischer Anisotropie verwendet.

Die erfindungsgemäßen flüssigkristallinen Dielektrika bestehen aus zwei oder mehr Komponenten, darunter mindestens eine der Formel (I). Die weiteren Komponenten sind vorzugsweise nematische oder nematogene Substanzen aus den Klassen der Azobenzole, Azoxybenzole, Biphenyle, Schiffschen Basen, insbesondere Benzyliden-Derivate, Phenylbenzoate, Phenylcyclohexane, gegebenenfalls halogenierten Stilbene, Diphenylacetylen-Derivate, Diphenylnitrone und substituierten Zimtsäuren. Die wichtigsten als derartige weitere Komponenten in Frage kommenden Verbindungen lassen sich durch die Formel (IV) charakterisieren:

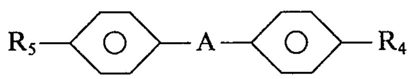 (IV)

worin

| | | |
|---|---|---|
| A | —CH=CH— | 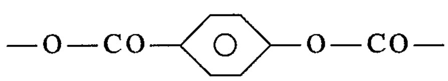 |
| | —CX'=CH— | 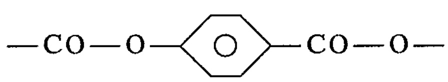 |
| | —CH=CX'— | 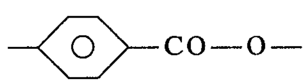 |
| | —C=C— | 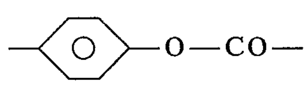 |
| | —N=N— | 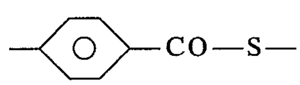 |
| | —N(O)=N— | 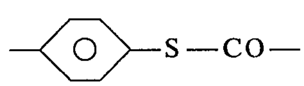 |
| | —N=N(O)— | —CH=N— |
| | —O—CO— | —N=CH— |

A —CO—O— —CH=N(O)—

—S—CO— —N(O)=CH—

—CO—S— oder eine C–C-Einfachbindung

bedeutet. Weitere mögliche Komponenten der erfindungsgemäßen Dielektrika sind solche Verbindungen der Formel (IV), in denen einer oder mehrere Phenylringe durch eine entsprechende Zahl von trans-Cyclohexylringen ersetzt sind; X′ bedeutet Halogen, vorzugsweise Cl oder –CN. $R_5$ und $R_4$ sind gleich oder verschieden und können Alkyl-, Alkoxy-, Alkanoyl-, Alkanoyloxy- oder Alkoxycarbonyloxyreste mit bis zu 18, vorzugsweise bis zu 8 C-Atomen bedeuten. Bei den meisten dieser Verbindungen sind $R_5$ und $R_4$ vorzugsweise verschieden, wobei einer der Reste meist eine Alkyl- oder Alkoxygruppe bedeutet. Aber auch eine große Zahl anderer Varianten der vorgesehenen Substituenten sind gebräuchlich. Viele solcher Substanzen sind im Handel erhältlich.

Die erfindungsgemäßen Dielektrika enthalten in der Regel mindestens 30, vorzugsweise 50–99, insbesondere 60–98 Gewichtsteile der Verbindungen der Formeln (I) und gegebenenfalls (IV). Hiervon entfallen bevorzugt mindestens 5 Gewichtsteile, meist auch 10 oder mehr Gewichtsteile auf eine oder mehrere Verbindungen der Formel (I). Es werden von der Erfindung auch solche flüssigkristallinen Dielektrika umfaßt, denen beispielsweise zu Dotierungszwecken nur weniger als 5 Gewichtsteile, zum Beispiel 0,1 bis 3 Gewichtsteile einer oder mehrerer Verbindungen der Formel (I) zugesetzt worden sind.

Die Herstellung der erfindungsgemäßen Dielektrika erfolgt in an sich üblicher Weise. In der Regel wird die gewünschte Menge einer oder mehrerer Verbindungen der Formel (I) in den weiteren Komponenten gelöst, zweckmäßig bei erhöhter Temperatur. Wenn dabei eine Temperatur oberhalb des Klärpunkts des Basismaterials gewählt wird, kann die Vollständigkeit des Lösevorgangs besonders leicht beobachtet werden.

Es ist jedoch auch möglich, Lösungen der Komponenten der Formeln (I) und (IV) in einem geeigneten organischen Lösungsmittel, zum Beispiel Aceton, Chloroform oder Methanol, zu mischen und das Lösungsmittel nach gründlicher Durchmischung wieder zu entfernen, beispielsweise durch Destillation unter vermindertem Druck. Selbstverständlich muß bei dieser Verfahrensweise darauf geachtet werden, daß durch das Lösungsmittel keine Verunreinigungen oder unerwünschten Dotierungsstoffe eingeschleppt werden.

Durch geeignete Zusätze können die flüssigkristallinen Dielektrika nach der Erfindung so modifiziert werden, daß sie in allen bisher bekanntgewordenen Arten von Flüssigkristallanzeigeelementen verwendet werden können. Derartige Zusätze sind dem Fachmann bekannt und sind in der einschlägigen Literatur ausführlich beschrieben. Beispielsweise können Substanzen zur Veränderung der dielektrischen Anisotropie, der Viskosität, der Leitfähigkeit und/oder der Orientierung der nematischen Phasen zugesetzt werden. Derartige Substanzen sind zum Beispiel in den DE-OS 2 209 127, 2 240 864, 2 321 632, 2 338 281 und 2 450 088 beschrieben.

Die folgenden Beispiele sollen die Erfindung erläutern. In den Beispielen bedeuten F. den Schmelzpunkt und K. den Klärpunkt einer flüssigkristallinen Substanz in Grad Celsius; Siedetemperaturen sind mit Kp. bezeichnet. Wenn nichts anderes angegeben ist, bedeuten Angaben von Teilen oder Prozent Gewichtsteile bzw. Gewichtsprozent.

## Beispiel 1

(a) In einer Suspension von 150 g wasserfreiem Aluminiumchlorid in 2000 ml Dichlormethan werden bei 10° unter Rühren 306 g 4-(4-n-Pentylcyclohexyl)-biphenyl gelöst und zu der Lösung unter Rühren im Lauf von 2 Stunden eine Lösung von 79 g Acetylchlorid in 250 ml Dichlormethan getropft. Das Reaktionsgemisch wird noch 16 Stunden gerührt und in eine Lösung von 300 ml konzentrierter Salzsäure in 2,5 l Eiswasser gegossen. Die organische Phase wird abgetrennt, mit Wasser und Natriumhydrogencarbonatlösung gewaschen, über Calciumchlorid getrocknet und eingedampft. Das zurückbleibende p-(4-n-Pentylcyclohexyl)-p′-acetylbiphenyl wird aus Äthanol umkristallisiert; F. 125°, Ausbeute 315 g.

(b) 175 g p-(4-n-Pentylcyclohexyl)-p′-acetylbiphenyl werden in 1,5 l Tetrahydrofuran gelöst und in Gegenwart von 10 g Palladium/Kohle (5% Pd) bei Raumtemperatur und Normaldruck 80 Stunden hydriert. Anschließend wird der Katalysator abfiltriert, das Filtrat eingedampft und das zurückbleibende p-(4-n-Pentylcyclohexyl)-p′-äthylbiphenyl aus Äthanol umkristallisiert; F. 34°, K. 164°, Ausbeute 149 g.

Analog werden hergestellt:

p-(4-Methylcyclohexyl)-p′-methylbiphenyl,
p-(4-Äthylcyclohexyl)-p′-methylbiphenyl,

p-(4-n-Propylcyclohexyl)-p'-methylbiphenyl,
p-(4-n-Butylcyclohexyl)-p'-methylbiphenyl,
p-(4-n-Pentylcyclohexyl)-p'-methylbiphenyl,
p-(4-n-Hexylcyclohexyl)-p'-methylbiphenyl,
p-(4-n-Heptylcyclohexyl)-p'-methylbiphenyl,
p-(4-n-Octylcyclohexyl)-p'-methylbiphenyl,
p-(4-n-Nonylcyclohexyl)-p'-methylbiphenyl,
p-(4-n-Decylcyclohexyl)-p'-methylbiphenyl,
p-(4-n-Undecylcyclohexyl)-p'-methylbiphenyl,
p-(4-n-Dodecylcyclohexyl)-p'-methylbiphenyl,
p-[4-(2-Methylpropyl)-cyclohexyl]-p'-methylbiphenyl,
p-[4-(2-Methylbutyl)-cyclohexyl]-p'-methylbiphenyl,
p-[4-(3-Methylbutyl)-cyclohexyl]-p'-methylbiphenyl,
p-[4-(2-Äthylhexyl)-cyclohexyl]-p'-methylbiphenyl.

p-(4-Methylcyclohexyl)-p'-äthylbiphenyl,
p-(4-Äthylcyclohexyl)-p'-äthylbiphenyl,
p-(4-n-Propylcyclohexyl)-p'-äthylbiphenyl,
p-(4-n-Butylcyclohexyl)-p'-äthylbiphenyl,
p-(4-n-Hexylcyclohexyl)-p'-äthylbiphenyl, F. 44°, K. 156°;
p-(4-n-Heptylcyclohexyl)-p'-äthylbiphenyl,
p-(4-n-Octylcyclohexyl)-p'-äthylbiphenyl,
p-(4-n-Nonylcyclohexyl)-p'-äthylbiphenyl,
p-(4-n-Decylcyclohexyl)-p'-äthylbiphenyl,
p-(4-n-Undecylcyclohexyl)-p'-äthylbiphenyl,
p-(4-n-Dodecylcyclohexyl)-p'-äthylbiphenyl,
p-[4-(2-Methylpropyl)-cyclohexyl]-p'-äthylbiphenyl,
p-[4-(2-Methylbutyl)-cyclohexyl]-p'-äthylbiphenyl,
p-[4-(3-Methylbutyl)-cyclohexyl]-p'-äthylbiphenyl,
p-[4-(2-Äthylhexyl)-cyclohexyl]-p'-äthylbiphenyl.

p-(4-Methylcyclohexyl)-p'-n-propylbiphenyl,
p-(4-Äthylcyclohexyl)-p'-n-propylbiphenyl,
p-(4-n-Propylcyclohexyl)-p'-n-propylbiphenyl,
p-(4-n-Butylcyclohexyl)-p'-n-propylbiphenyl,
p-(4-n-Pentylcyclohexyl)-p'-n-propylbiphenyl, F. 14°, K. 158°;
p-(4-n-Hexylcyclohexyl)-p'-n-propylbiphenyl,
p-(4-n-Heptylcyclohexyl)-p'-n-propylbiphenyl,
p-(4-n-Octylcyclohexyl)-p'-n-propylbiphenyl,
p-(4-n-Nonylcyclohexyl)-p'-n-propylbiphenyl,
p-(4-n-Decylcyclohexyl)-p'-n-propylbiphenyl,
p-(4-n-Undecylcyclohexyl)-p'-n-propylbiphenyl,
p-(4-n-Dodecylcyclohexyl)-p'-n-propylbiphenyl,
p-[4-(2-Methylpropyl)-cyclohexyl]-p'-n-propylbiphenyl,
p-[4-(2-Methylbutyl)-cyclohexyl]-p'-n-propylbiphenyl,
p-[4-(3-Methylbutyl)-cyclohexyl]-p'-n-propylbiphenyl,
p-[4-(2-Äthylhexyl)-cyclohexyl]-p'-n-propylbiphenyl.

p-(4-Methylcyclohexyl)-p'-n-butylbiphenyl,
p-(4-Äthylcyclohexyl)-p'-n-butylbiphenyl,
p-(4-n-Propylcyclohexyl)-p'-n-butylbiphenyl,
p-(4-n-Butylcyclohexyl)-p'-n-butylbiphenyl,
p-(4-n-Pentylcyclohexyl)-p'-n-butylbiphenyl, F. 20°, K. 170,5°;
p-(4-n-Hexylcyclohexyl)-p'-n-butylbiphenyl,
p-(4-n-Heptylcyclohexyl)-p'-n-butylbiphenyl,
p-(4-n-Octylcyclohexyl)-p'-n-butylbiphenyl,
p-(4-n-Nonylcyclohexyl)-p'-n-butylbiphenyl,
p-(4-n-Decylcyclohexyl)-p'-n-butylbiphenyl,
p-(4-n-Undecylcyclohexyl)-p'-n-butylbiphenyl,
p-(4-n-Dodecylcyclohexyl)-p'-n-butylbiphenyl,
p-[4-(2-Methylpropyl)-cyclohexyl]-p'-n-butylbiphenyl,
p-[4-(2-Methylbutyl)-cyclohexyl]-p'-n-butylbiphenyl,
p-[4-(3-Methylbutyl)-cyclohexyl]-p'-n-butylbiphenyl,
p-[4-(2-Äthylhexyl)-cyclohexyl]-p'-n-butylbiphenyl.

p-(4-Methylcyclohexyl)-p'-n-pentylbiphenyl,
p-(4-Äthylcyclohexyl)-p'-n-pentylbiphenyl,
p-(4-n-Propylcyclohexyl)-p'-n-pentylbiphenyl,
p-(4-n-Butylcyclohexyl)-p'-n-pentylbiphenyl,
p-(4-n-Pentylcyclohexyl)-p'-n-pentylbiphenyl, f. 13°, K. 170°;
p-(4-n-Hexylcyclohexyl)-p'-n-pentylbiphenyl,
p-(4-n-Heptylcyclohexyl)-p'-n-pentylbiphenyl,
p-(4-n-Octylcyclohexyl)-p'-n-pentylbiphenyl,
p-(4-n-Nonylcyclohexyl)-p'-n-pentylbiphenyl,
p-(4-n-Decylcyclohexyl)-p'-n-pentylbiphenyl,
p-(4-n-Undecylcyclohexyl)-p'-n-pentylbiphenyl,
p-(4-n-Dodecylcyclohexyl)-p'-n-pentylbiphenyl,
p-[4-(2-Methylpropyl)-cyclohexyl]-p'-n-pentylbiphenyl,
p-[4-(2-Methylbutyl)-cyclohexyl]-p'-n-pentylbiphenyl,
p-[4-(3-Methylbutyl)-cyclohexyl]-p'-n-pentylbiphenyl,
p-[4-(2-Äthylhexyl)-cyclohexyl]-p'-n-pentylbiphenyl.

p-(4-Methylcyclohexyl)-p'-n-hexylbiphenyl,
p-(4-Äthylcyclohexyl)-p'-n-hexylbiphenyl,
p-(4-n-Propylcyclohexyl)-p'-n-hexylbiphenyl,
p-(4-n-Butylcyclohexyl)-p'-n-hexylbiphenyl,
p-(4-n-Pentylcyclohexyl)-p'-n-hexylbiphenyl,
p-(4-n-Hexylcyclohexyl)-p'-n-hexylbiphenyl,
p-(4-n-Heptylcyclohexyl)-p'-n-hexylbiphenyl,
p-(4-n-Octylcyclohexyl)-p'-n-hexylbiphenyl,
p-(4-n-Nonylcyclohexyl)-p'-n-hexylbiphenyl,
p-(4-n-Decylcyclohexyl)-p'-n-hexylbiphenyl,
p-[4-(2-Methylpropyl)-cyclohexyl]-p'-n-hexylbiphenyl,
p-[4-(2-Methylbutyl)-cyclohexyl]-p'-n-hexylbiphenyl,
p-[4-(3-Methylbutyl)-cyclohexyl]-p'-n-hexylbiphenyl,
p-[4-(2-Äthylhexyl)-cyclohexyl]-p'-n-hexylbiphenyl.

p-(4-Methylcyclohexyl)-p'-n-heptylbiphenyl,
p-(4-Äthylcyclohexyl)-p'-n-heptylbiphenyl,
p-(4-n-Propylcyclohexyl)-p'-n-heptylbiphenyl,
p-(4-n-Butylcyclohexyl)-p'-n-heptylbiphenyl,
p-(4-n-Pentylcyclohexyl)-p'-n-heptylbiphenyl,
p-(4-n-Hexylcyclohexyl)-p'-n-heptylbiphenyl,
p-(4-n-Heptylcyclohexyl)-p'-n-heptylbiphenyl,
p-(4-n-Octylcyclohexyl)-p'-n-heptylbiphenyl,
p-(4-n-Nonylcyclohexyl)-p'-n-heptylbiphenyl,
p-(4-n-Decylcyclohexyl)-p'-n-heptylbiphenyl,
p-[4-(2-Methylpropyl)-cyclohexyl]-p'-n-heptylbiphenyl,
p-[4-(2-Methylbutyl)-cyclohexyl]-p'-n-heptylbiphenyl,
p-[4-(3-Methylbutyl)-cyclohexyl]-p'-n-heptylbiphenyl,
p-[4-(2-Äthylhexyl)-cyclohexyl]-p'-n-heptylbiphenyl.

p-(4-Methylcyclohexyl)-p'-n-octylbiphenyl,
p-(4-Äthylcyclohexyl)-p'-n-octylbiphenyl,
p-(4-n-Propylcyclohexyl)-p'-n-octylbiphenyl,
p-(4-n-Butylcyclohexyl)-p'-n-octylbiphenyl,
p-(4-n-Pentylcyclohexyl)-p'-n-octylbiphenyl,
p-(4-n-Hexylcyclohexyl)-p'-n-octylbiphenyl,
p-(4-n-Heptylcyclohexyl)-p'-n-octylbiphenyl,
p-(4-n-Octylcyclohexyl)-p'-n-octylbiphenyl,
p-[4-(2-Methylpropyl)-cyclohexyl]-p'-n-octylbiphenyl,
p-[4-(2-Methylbutyl)-cyclohexyl]-p'-n-octylbiphenyl,
p-[4-(3-Methylbutyl)-cyclohexyl]-p'-n-octylbiphenyl,
p-[4-(2-Äthylhexyl)-cyclohexyl]-p'-n-octylbiphenyl.

p-(4-Methylcyclohexyl)-p'-n-nonylbiphenyl,
p-(4-Äthylcyclohexyl)-p'-n-nonylbiphenyl,
p-(4-n-Propylcyclohexyl)-p'-n-nonylbiphenyl,
p-(4-n-Butylcyclohexyl)-p'-n-nonylbiphenyl,
p-(4-n-Pentylcyclohexyl)-p'-n-nonylbiphenyl,

p-(4-n-Hexylcyclohexyl)-p'-n-nonylbiphenyl,
p-(4-n-Heptylcyclohexyl)-p'-n-nonylbiphenyl,
p-[4-(2-Methylpropyl)-cyclohexyl]-p'-n-nonylbiphenyl,
p-[4-(2-Methylbutyl)-cyclohexyl]-p'-n-nonylbiphenyl,
p-[4-(3-Methylbutyl)-cyclohexyl]-p'-n-nonylbiphenyl,
p-[4-(2-Äthylhexyl)-cyclohexyl]-p'-n-nonylbiphenyl.

p-(4-Methylcyclohexyl)-p'-n-decylbiphenyl,
p-(4-Äthylcyclohexyl)-p'-n-decylbiphenyl,
p-(4-n-Propylcyclohexyl)-p'-n-decylbiphenyl,
p-(4-n-Butylcyclohexyl)-p'-n-decylbiphenyl,
p-(4-n-Pentylcyclohexyl)-p'-n-decylbiphenyl,
p-(4-n-Hexylcyclohexyl)-p'-n-decylbiphenyl,
p-(4-n-Heptylcyclohexyl)-p'-n-decylbiphenyl,
p-(4-n-Octylcyclohexyl)-p'-n-decylbiphenyl,
p-[4-(2-Methylpropyl)-cyclohexyl]-p'-n-decylbiphenyl,
p-[4-(2-Methylbutyl)-cyclohexyl]-p'-n-decylbiphenyl,
p-[4-(3-Methylbutyl)-cyclohexyl]-p'-n-decylbiphenyl,
p-[4-(2-Äthylhexyl)-cyclohexyl]-p'-n-decylbiphenyl.

p-(4-Methylcyclohexyl)-p'-n-undecylbiphenyl,
p-(4-Äthylcyclohexyl)-p'-n-undecylbiphenyl,
p-(4-n-Propylcyclohexyl)-p'-n-undecylbiphenyl,
p-(4-n-Butylcyclohexyl)-p'-n-undecylbiphenyl,
p-(4-n-Pentylcyclohexyl)-p'-n-undecylbiphenyl,
p-(4-n-Hexylcyclohexyl)-p'-n-undecylbiphenyl,
p-(4-n-Heptylcyclohexyl)-p'-n-undecylbiphenyl,
p-(4-n-Octylcyclohexyl)-p'-n-undecylbiphenyl,
p-[4-(2-Methylpropyl)-cyclohexyl]-p'-n-undecylbiphenyl,
p-[4-(2-Methylbutyl)-cyclohexyl]-p'-n-undecylbiphenyl,
p-[4-(3-Methylbutyl)-cyclohexyl]-p'-n-undecylbiphenyl,
p-[4-(2-Äthylhexyl)-cyclohexyl]-p'-n-undecylbiphenyl.

p-(4-Methylcyclohexyl)-p'-n-dodecylbiphenyl,
p-(4-Äthylcyclohexyl)-p'-n-dodecylbiphenyl,
p-(4-n-Propylcyclohexyl)-p'-n-dodecylbiphenyl,
p-(4-n-Butylcyclohexyl)-p'-n-dodecylbiphenyl,
p-(4-n-Pentylcyclohexyl)-p'-n-dodecylbiphenyl,
p-(4-n-Hexylcyclohexyl)-p'-n-dodecylbiphenyl,
p-(4-n-Heptylcyclohexyl)-p'-n-dodecylbiphenyl,
p-(4-n-Octylcyclohexyl)-p'-n-dodecylbiphenyl,
p-[4-(2-Methylpropyl)-cyclohexyl]-p'-n-dodecylbiphenyl,
p-[4-(2-Methylbutyl)-cyclohexyl]-p'-n-dodecylbiphenyl,
p-[4-(3-Methylbutyl)-cyclohexyl]-p'-n-dodecylbiphenyl,
p-[4-(2-Äthylhexyl)-cyclohexyl]-p'-n-dodecylbiphenyl.

p-(4-Methylcyclohexyl)-p'-(2-methylbutyl)-biphenyl,
p-(4-Äthylcyclohexyl)-p'-(2-methylbutyl)-biphenyl,
p-(4-n-Propylcyclohexyl)-p'-(2-methylbutyl)-biphenyl,
p-(4-n-Butylcyclohexyl)-p'-(2-methylbutyl)-biphenyl,
p-(4-n-Pentylcyclohexyl)-p'-(2-methylbutyl)-biphenyl, F. 50°, K. 88°;
p-(4-n-Hexylcyclohexyl)-p'-(2-methylbutyl)-biphenyl,
p-(4-n-Heptylcyclohexyl)-p'-(2-methylbutyl)-biphenyl,
p-(4-n-Octylcyclohexyl)-p'-(2-methylbutyl)-biphenyl,
p-(4-n-Nonylcyclohexyl)-p'-(2-methylbutyl)-biphenyl,
p-(4-n-Decylcyclohexyl)-p'-(2-methylbutyl)-biphenyl,
p-(4-n-Undecylcyclohexyl)-p'-(2-methylbutyl)-biphenyl,
p-(4-n-Dodecylcyclohexyl)-p'-(2-methylbutyl)-biphenyl.

p-(4-Methylcyclohexyl)-p'-(2-methylpentyl)-biphenyl,
p-(4-Äthylcyclohexyl)-p'-(2-methylpentyl)-biphenyl,
p-(4-n-Propylcyclohexyl)-p'-(2-methylpentyl)-biphenyl,
p-(4-n-Butylcyclohexyl)-p'-(2-methylpentyl)-biphenyl,
p-(4-n-Pentylcyclohexyl)-p'-(2-methylpentyl)-biphenyl,
p-(4-n-Hexylcyclohexyl)-p'-(2-methylpentyl)-biphenyl,

p-(4-n-Heptylcyclohexyl)-p'-(2-methylpentyl)-biphenyl,
p-(4-n-Octylcyclohexyl)-p'-(2-methylpentyl)-biphenyl,
p-(4-n-Nonylcyclohexyl)-p'-(2-methylpentyl)-biphenyl,
p-(4-n-Decylcyclohexyl)-p'-(2-methylpentyl)-biphenyl,
p-(4-n-Undecylcyclohexyl)-p'-(2-methylpentyl)-biphenyl,
p-(4-n-Dodecylcyclohexyl)-p'-(2-methylpentyl)-biphenyl.

p-(4-Methylcyclohexyl)-p'-(2-äthylhexyl)-biphenyl,
p-(4-Äthylcyclohexyl)-p'-(2-äthylhexyl)-biphenyl,
p-(4-n-Propylcyclohexyl)-p'-(2-äthylhexyl)-biphenyl,
p-(4-n-Butylcyclohexyl)-p'-(2-äthylhexyl)-biphenyl,
p-(4-n-Pentylcyclohexyl)-p'-(2-äthylhexyl)-biphenyl,
p-(4-n-Hexylcyclohexyl)-p'-(2-äthylhexyl)-biphenyl,
p-(4-n-Heptylcyclohexyl)-p'-(2-äthylhexyl)-biphenyl,
p-(4-n-Octylcyclohexyl)-p'-(2-äthylhexyl)-biphenyl,
p-(4-n-Nonylcyclohexyl)-p'-(2-äthylhexyl)-biphenyl,
p-(4-n-Decylcyclohexyl)-p'-(2-äthylhexyl)-biphenyl,
p-(4-n-Undecylcyclohexyl)-p'-(2-äthylhexyl)-biphenyl,
p-(4-n-Dodecylcyclohexyl)-p'-(2-äthylhexyl)-biphenyl.

Beispiel 2

Eine Suspension von 35 g p-(4-n-Pentylcyclohexyl)-p'-acetylbiphenyl in 200 ml 98%iger Ameisensäure wird mit einer Mischung von 20 ml 33%igem Wasserstoffperoxid und 50 ml 98%iger Ameisensäure versetzt und bei Raumtemperatur 72 Stunden gerührt. Das Reaktionsgemisch wird auf 1000 g Eis gegossen und zweimal mit je 500 ml Dichlormethan extrahiert. Das nach Abdampfen des Lösungsmittels zurückbleibende p-(4-n-Pentylcyclohexyl)-p'-acetoxybiphenyl wird aus Äthanol umkristallisiert; F. 77°, K. 215°.

Analog werden hergestellt:

p-(4-Methylcyclohexyl)-p'-acetoxybiphenyl,
p-(4-Äthylcyclohexyl)-p'-acetoxybiphenyl,
p-(4-n-Propylcyclohexyl)-p'-acetoxybiphenyl,
p-(4-n-Butylcyclohexyl)-p'-acetoxybiphenyl,
p-(4-n-Hexylcyclohexyl)-p'-acetoxybiphenyl,
p-(4-n-Heptylcyclohexyl)-p'-acetoxybiphenyl,
p-(4-n-Octylcyclohexyl)-p'-acetoxybiphenyl,
p-(4-n-Nonylcyclohexyl)-p'-acetoxybiphenyl,
p-(4-n-Decylcyclohexyl)-p'-acetoxybiphenyl,
p-(4-n-Undecylcyclohexyl)-p'-acetoxybiphenyl,
p-(4-n-Dodecylcyclohexyl)-p'-acetoxybiphenyl,
p-[4-(2-Methylpropyl)-cyclohexyl]-p'-acetoxybiphenyl,
p-[4-(2-Methylbutyl)-cyclohexyl]-p'-acetoxybiphenyl,
p-[4-(3-Methylbutyl)-cyclohexyl]-p'-acetoxybiphenyl,
p-[4-(2-Äthylhexyl)-cyclohexyl]-p'-acetoxybiphenyl.

Beispiel 3

a) 46 g p-(4-n-Pentylcyclohexyl)-p'-acetoxybiphenyl werden mit 20 g Natriumhydroxid in 250 ml Wasser und 85 ml Äthanol suspendiert. Die Suspension wird 2 Stunden unter Rückfluß zum Sieden erhitzt und anschließend mit Salzsäure angesäuert. Das ausgefallene p-(4-n-Pentylcyclohexyl)-p'-hydroxybiphenyl wird abfiltriert und mit Wasser neutral gewaschen; F. 202°.
b) Zu 9,0 g p-(4-n-Pentylcyclohexyl)-p'-hydroxybiphenyl wird im Laufe von 10 Minuten eine Lösung von 3,4 g Valeriansäurechlorid in 20 ml Toluol so zugetropft, daß die Temperatur 30° nicht übersteigt. Anschließend werden 2,5 g Pyridin zugefügt und die Mischung 12 Stunden bei 20° gerührt. Danach wird das Reaktionsgemisch in 500 ml Wasser eingegossen, die organische Phase abgetrennt, mit Wasser neutral gewaschen und eingedampft. Das zurückbleibende p-(4-n-Pentylcyclohexyl)-p'-n-pentanoyloxybiphenyl wird aus Äthanol umkristallisiert; F. 69°, K. 199°.

Analog werden hergestellt:

p-(4-Methylcyclohexyl)-p'-propionyloxybiphenyl,
p-(4-Äthylcyclohexyl)-p'-propionyloxybiphenyl,
p-(4-n-Propylcyclohexyl)-p'-propionyloxybiphenyl,

p-(4-n-Butylcyclohexyl)-p'-propionyloxybiphenyl,
p-(4-n-Pentylcyclohexyl)-p'-propionyloxybiphenyl,
p-(4-n-Hexylcyclohexyl)-p'-propionyloxybiphenyl,
p-(4-n-Heptylcyclohexyl)-p'-propionyloxybiphenyl,
p-(4-n-Octylcyclohexyl)-p'-propionyloxybiphenyl,
p-(4-n-Nonylcyclohexyl)-p'-propionyloxybiphenyl,
p-(4-n-Decylcyclohexyl)-p'-propionyloxybiphenyl,
p-(4-n-Undecylcyclohexyl)-p'-propionyloxybiphenyl,
p-(4-n-Dodecylcyclohexyl)-p'-propionyloxybiphenyl,
p-[4-(2-Methylpropyl)-cyclohexyl]-p'-propionyloxybiphenyl,
p-[4-(2-Methylbutyl)-cyclohexyl]-p'-propionyloxybiphenyl,
p-[4-(3-Methylbutyl)-cyclohexyl]-p'-propionyloxybiphenyl,
p-[4-(2-Äthylhexyl)-cyclohexyl]-p'-propionyloxybiphenyl.

p-(4-Methylcyclohexyl)-p'-butyryloxybiphenyl,
p-(4-Äthylcyclohexyl)-p'-butyryloxybiphenyl,
p-(4-n-Propylcyclohexyl)-p'-butyryloxybiphenyl,
p-(4-n-Butylcyclohexyl)-p'-butyryloxybiphenyl,
p-(4-n-Pentylcyclohexyl)-p'-butyryloxybiphenyl,
p-(4-n-Hexylcyclohexyl)-p'-butyryloxybiphenyl,
p-(4-n-Heptylcyclohexyl)-p'-butyryloxybiphenyl,
p-(4-n-Octylcyclohexyl)-p'-butyryloxybiphenyl,
p-(4-n-Nonylcyclohexyl)-p'-butyryloxybiphenyl,
p-(4-n-Decylcyclohexyl)-p'-butyryloxybiphenyl,
p-(4-n-Undecylcyclohexyl)-p'-butyryloxybiphenyl,
p-(4-n-Dodecylcyclohexyl)-p'-butyryloxybiphenyl,
p-[4-(2-Methylpropyl)-cyclohexyl]-p'-butyryloxybiphenyl,
p-[4-(2-Methylbutyl)-cyclohexyl]-p'-butyryloxybiphenyl,
p-[4-(3-Methylbutyl)-cyclohexyl]-p'-butyryloxybiphenyl,
p-[4-(2-Äthylhexyl)-cyclohexyl]-p'-butyryloxybiphenyl.

p-(4-Methylcyclohexyl)-p'-n-pentanoyloxybiphenyl,
p-(4-Äthylcyclohexyl)-p'-n-pentanoyloxybiphenyl,
p-(4-n-Propylcyclohexyl)-p'-n-pentanoyloxybiphenyl,
p-(4-n-Butylcyclohexyl)-p'-n-pentanoyloxybiphenyl,
p-(4-n-Hexylcyclohexyl)-p'-n-pentanoyloxybiphenyl,
p-(4-n-Heptylcyclohexyl)-p'-n-pentanoyloxybiphenyl,
p-(4-n-Octylcyclohexyl)-p'-n-pentanoyloxybiphenyl,
p-(4-n-Nonylcyclohexyl)-p'-n-pentanoyloxybiphenyl,
p-(4-n-Decylcyclohexyl)-p'-n-pentanoyloxybiphenyl,
p-(4-n-Undecylcyclohexyl)-p'-n-pentanoyloxybiphenyl,
p-(4-n-Dodecylcyclohexyl)-p'-n-pentanoyloxybiphenyl,
p-[4-(2-Methylpropyl)-cyclohexyl]-p'-n-pentanoyloxybiphenyl,
p-[4-(2-Methylbutyl)-cyclohexyl]-p'-n-pentanoyloxybiphenyl,
p-[4-(3-Methylbutyl)-cyclohexyl]-p'-n-pentanoyloxybiphenyl,
p-[4-(2-Äthylhexyl)-cyclohexyl]-p'-n-pentanoyloxybiphenyl.

p-(4-Methylcyclohexyl)-p'-n-hexanoyloxybiphenyl,
p-(4-Äthylcyclohexyl)-p'-n-hexanoyloxybiphenyl,
p-(4-n-Propylcyclohexyl)-p'-n-hexanoyloxybiphenyl,
p-(4-n-Butylcyclohexyl)-p'-n-hexanoyloxybiphenyl,
p-(4-n-Pentylcyclohexyl)-p'-n-hexanoyloxybiphenyl,
p-(4-n-Hexylcyclohexyl)-p'-n-hexanoyloxybiphenyl,
p-(4-n-Heptylcyclohexyl)-p'-n-hexanoyloxybiphenyl,
p-(4-n-Octylcyclohexyl)-p'-n-hexanoyloxybiphenyl,
p-(4-n-Nonylcyclohexyl)-p'-n-hexanoyloxybiphenyl,
p-(4-n-Decylcyclohexyl)-p'-n-hexanoyloxybiphenyl,
p-(4-n-Undecylcyclohexyl)-p'-n-hexanoyloxybiphenyl,
p-(4-n-Dodecylcyclohexyl)-p'-n-hexanoyloxybiphenyl,
p-[4-(2-Methylpropyl)-cyclohexyl]-p'-n-hexanoyloxybiphenyl,
p-[4-(2-Methylbutyl)-cyclohexyl]-p'-n-hexanoyloxybiphenyl,
p-[4-(3-Methylbutyl)-cyclohexyl]-p'-n-hexanoyloxybiphenyl,
p-[4-(2-Äthylhexyl)-cyclohexyl]-p'-n-hexanoyloxybiphenyl.

p-(4-Methylcyclohexyl)-p'-n-heptanoyloxybiphenyl,
p-(4-Äthylcyclohexyl)-p'-n-heptanoyloxybiphenyl,
p-(4-n-Propylcyclohexyl)-p'-n-heptanoyloxybiphenyl,
p-(4-n-Butylcyclohexyl)-p'-n-heptanoyloxybiphenyl,
p-(4-n-Pentylcyclohexyl)-p'-n-heptanoyloxybiphenyl,
p-(4-n-Hexylcyclohexyl)-p'-n-heptanoyloxybiphenyl,
p-(4-n-Heptylcyclohexyl)-p'-n-heptanoyloxybiphenyl,
p-(4-n-Octylcyclohexyl)-p'-n-heptanoyloxybiphenyl,
p-(4-n-Nonylcyclohexyl)-p'-n-heptanoyloxybiphenyl,
p-(4-n-Decylcyclohexyl)-p'-n-heptanoyloxybiphenyl,
p-[4-(2-Methylpropyl)-cyclohexyl]-p'-n-heptanoyloxybiphenyl,
p-[4-(2-Methylbutyl)-cyclohexyl]-p'-n-heptanoyloxybiphenyl,
p-[4-(3-Methylbutyl)-cyclohexyl]-p'-n-heptanoyloxybiphenyl,
p-[4-(2-Äthylhexyl)-cyclohexyl]-p'-n-heptanoyloxybiphenyl.

p-(4-Methylcyclohexyl)-p'-n-octanoyloxybiphenyl,
p-(4-Äthylcyclohexyl)-p'-n-octanoyloxybiphenyl,
p-(4-n-Propylcyclohexyl)-p'-n-octanoyloxybiphenyl,
p-(4-n-Butylcyclohexyl)-p'-n-octanoyloxybiphenyl,
p-(4-n-Pentylcyclohexyl)-p'-n-octanoyloxybiphenyl,
p-(4-n-Hexylcyclohexyl)-p'-n-octanoyloxybiphenyl,
p-(4-n-Heptylcyclohexyl)-p'-n-octanoyloxybiphenyl,
p-(4-n-Octylcyclohexyl)-p'-n-octanoyloxybiphenyl,
p-[4-(2-Methylpropyl)-cyclohexyl]-p'-n-octanoyloxybiphenyl,
p-[4-(2-Methylbutyl)-cyclohexyl]-p'-n-octanoyloxybiphenyl,
p-[4-(3-Methylbutyl)-cyclohexyl]-p'-n-octanoyloxybiphenyl,
p-[4-(2-Äthylhexyl)-cyclohexyl]-p'-n-octanoyloxybiphenyl.

p-(4-Methylcyclohexyl)-p'-trifluoracetoxybiphenyl,
p-(4-Äthylcyclohexyl)-p'-trifluoracetoxybiphenyl,
p-(4-n-Propylcyclohexyl)-p'-trifluoracetoxybiphenyl,
p-(4-n-Butylcyclohexyl)-p'-trifluoracetoxybiphenyl,
p-(4-n-Pentylcyclohexyl)-p'-trifluoracetoxybiphenyl,
p-(4-n-Hexylcyclohexyl)-p'-trifluoracetoxybiphenyl,
p-(4-n-Heptylcyclohexyl)-p'-trifluoracetoxybiphenyl,
p-(4-n-Octylcyclohexyl)-p'-trifluoracetoxybiphenyl,
p-(4-n-Nonylcyclohexyl)-p'-trifluoracetoxybiphenyl,
p-(4-n-Decylcyclohexyl)-p'-trifluoracetoxybiphenyl,
p-(4-n-Undecylcyclohexyl)-p'-trifluoracetoxybiphenyl,
p-(4-n-Dodecylcyclohexyl)-p'-trifluoracetoxybiphenyl,
p-[4-(2-Methylpropyl)-cyclohexyl]-p'-trifluoracetoxybiphenyl,
p-[4-(2-Methylbutyl)-cyclohexyl]-p'-trifluoracetoxybiphenyl,
p-[4-(3-Methylbutyl)-cyclohexyl]-p'-trifluoracetoxybiphenyl,
p-[4-(2-Äthylhexyl)-cyclohexyl]-p'-trifluoracetoxybiphenyl.

p-(4-Methylcyclohexyl)-p'-heptafluorbutyryloxybiphenyl,
p-(4-Äthylcyclohexyl)-p'-heptafluorbutyryloxybiphenyl,
p-(4-n-Propylcyclohexyl)-p'-heptafluorbutyryloxybiphenyl,
p-(4-n-Butylcyclohexyl)-p'-heptafluorbutyryloxybiphenyl,
p-(4-n-Pentylcyclohexyl)-p'-heptafluorbutyryloxybiphenyl,
p-(4-n-Hexylcyclohexyl)-p'-heptafluorbutyryloxybiphenyl,
p-(4-n-Heptylcyclohexyl)-p'-heptafluorbutyryloxybiphenyl,
p-(4-n-Octylcyclohexyl)-p'-heptafluorbutyryloxybiphenyl,
p-(4-n-Nonylcyclohexyl)-p'-heptafluorbutyryloxybiphenyl,
p-(4-n-Decylcyclohexyl)-p'-heptafluorbutyryloxybiphenyl,
p-[4-(2-Methylpropyl)-cyclohexyl]-p'-heptafluorbutyryloxybiphenyl,
p-[4-(2-Methylbutyl)-cyclohexyl]-p'-heptafluorbutyryloxybiphenyl,
p-[4-(3-Methylbutyl)-cyclohexyl]-p'-heptafluorbutyryloxybiphenyl,
p-[4-(2-Äthylhexyl)-cyclohexyl]-p'-heptafluorbutyryloxybiphenyl.

## Beispiel 4

Eine Lösung von 32 g p-(4-Pentylcyclohexyl)-p'-hydroxybiphenyl in 250 ml Aceton wird mit 31 g n-Pentylbromid in Gegenwart von 70 g wasserfreiem Kaliumcarbonat 24 Stunden unter Rückfluß zum

Sieden erhitzt. Nach Abkühlen wird das Reaktionsgemisch filtriert, das Filtrat eingedampft und das zurückbleibende p-(4-n-Pentylcyclohexyl)-p'-n-pentyloxybiphenyl aus Äthanol umkristallisiert; F. 42°, K. 183°, Ausbeute 37 g.

Analog werden hergestellt:

p-(4-Methylcyclohexyl)-p'-methoxy-biphenyl,
p-(4-Äthylcyclohexyl)-p'-methoxy-biphenyl,
p-(4-n-Propylcyclohexyl)-p'-methoxy-biphenyl,
p-(4-n-Butylcyclohexyl)-p'-methoxy-biphenyl,
p-(4-n-Pentylcyclohexyl)-p'-methoxy-biphenyl, F. 80°, K. 165°;
p-(4-n-Hexylcyclohexyl)-p'-methoxy-biphenyl,
p-(4-n-Heptylcyclohexyl)-p'-methoxy-biphenyl,
p-(4-n-Octylcyclohexyl)-p'-methoxy-biphenyl,
p-(4-n-Nonylcyclohexyl)-p'-methoxy-biphenyl,
p-(4-n-Decylcyclohexyl)-p'-methoxy-biphenyl,
p-(4-n-Undecylcyclohexyl)-p'-methoxy-biphenyl,
p-(4-n-Dodecylcyclohexyl)-p'-methoxy-biphenyl,
p-[4-(2-Methylpropyl)-cyclohexyl]-p'-methoxy-biphenyl,
p-[4-(2-Methylbutyl)-cyclohexyl]-p'-methoxy-biphenyl,
p-[4-(3-Methylbutyl)-cyclohexyl]-p'-methoxy-biphenyl,
p-[4-(2-Äthylhexyl)-cyclohexyl]-p'-methoxy-biphenyl.

p-(4-Methylcyclohexyl)-p'-äthoxy-biphenyl,
p-(4-Äthylcyclohexyl)-p'-äthoxy-biphenyl,
p-(4-n-Propylcyclohexyl)-p'-äthoxy-biphenyl,
p-(4-n-Butylcyclohexyl)-p'-äthoxy-biphenyl,
p-(4-n-Pentylcyclohexyl)-p'-äthoxy-biphenyl, F. 89°, K. 148°;
p-(4-n-Hexylcyclohexyl)-p'-äthoxy-biphenyl,
p-(4-n-Heptylcyclohexyl)-p'-äthoxy-biphenyl,
p-(4-n-Octylcyclohexyl)-p'-äthoxy-biphenyl,
p-(4-n-Nonylcyclohexyl)-p'-äthoxy-biphenyl,
p-(4-n-Decylcyclohexyl)-p'-äthoxy-biphenyl,
p-(4-n-Undecylcyclohexyl)-p'-äthoxy-biphenyl,
p-(4-n-Dodecylcyclohexyl)-p'-äthoxy-biphenyl,
p-[4-(2-Methylpropyl)-cyclohexyl]-p'-äthoxy-biphenyl,
p-[4-(2-Methylbutyl)-cyclohexyl]-p'-äthoxy-biphenyl,
p-[4-(3-Methylbutyl)-cyclohexyl]-p'-äthoxy-biphenyl,
p-[4-(2-Äthylhexyl)-cyclohexyl]-p'-äthoxy-biphenyl.

p-(4-Methylcyclohexyl)-p'-n-propyloxybiphenyl,
p-(4-Äthylcyclohexyl)-p'-n-propyloxybiphenyl,
p-(4-n-Propylcyclohexyl)-p'-n-propyloxybiphenyl,
p-(4-n-Butylcyclohexyl)-p'-n-propyloxybiphenyl,
p-(4-n-Pentylcyclohexyl)-p'-n-propyloxybiphenyl,
p-(4-n-Hexylcyclohexyl)-p'-n-propyloxybiphenyl,
p-(4-n-Heptylcyclohexyl)-p'-n-propyloxybiphenyl,
p-(4-n-Octylcyclohexyl)-p'-n-propyloxybiphenyl,
p-(4-n-Nonylcyclohexyl)-p'-n-propyloxybiphenyl,
p-(4-n-Decylcyclohexyl)-p'-n-propyloxybiphenyl,
p-(4-n-Undecylcyclohexyl)-p'-n-propyloxybiphenyl,
p-(4-n-Dodecylcyclohexyl)-p'-n-propyloxybiphenyl,
p-[4-(2-Methylpropyl)-cyclohexyl]-p'-n-propyloxybiphenyl,
p-[4-(2-Methylbutyl)-cyclohexyl]-p'-n-propyloxybiphenyl,
p-[4-(3-Methylbutyl)-cyclohexyl]-p'-n-propyloxybiphenyl,
p-[4-(2-Äthylhexyl)-cyclohexyl]-p'-n-propyloxybiphenyl.

p-(4-Methylcyclohexyl)-p'-n-butyloxybiphenyl,
p-(4-Äthylcyclohexyl)-p'-n-butyloxybiphenyl,
p-(4-n-Propylcyclohexyl)-p'-n-butyloxybiphenyl,
p-(4-n-Butylcyclohexyl)-p'-n-butyloxybiphenyl,
p-(4-n-Pentylcyclohexyl)-p'-n-butyloxybiphenyl,
p-(4-n-Hexylcyclohexyl)-p'-n-butyloxybiphenyl,
p-(4-n-Heptylcyclohexyl)-p'-n-butyloxybiphenyl,
p-(4-n-Octylcyclohexyl)-p'-n-butyloxybiphenyl,
p-(4-n-Nonylcyclohexyl)-p'-n-butyloxybiphenyl,

p-(4-n-Decylcyclohexyl)-p'-n-butyloxybiphenyl,
p-[4-(2-Methylpropyl)-cyclohexyl]-p'-n-butyloxybiphenyl,
p-[4-(2-Methylbutyl)-cyclohexyl]-p'-n-butyloxybiphenyl,
p-[4-(3-Methylbutyl)-cyclohexyl]-p'-n-butyloxybiphenyl,
p-[4-(2-Äthylhexyl)-cyclohexyl]-p'-n-butyloxybiphenyl.

p-(4-Methylcyclohexyl)-p'-n-pentyloxybiphenyl,
p-(4-Äthylcyclohexyl)-p'-n-pentyloxybiphenyl,
p-(4-n-Propylcyclohexyl)-p'-n-pentyloxybiphenyl,
p-(4-n-Butylcyclohexyl)-p'-n-pentyloxybiphenyl,
p-(4-n-Hexylcyclohexyl)-p'-n-pentyloxybiphenyl,
p-(4-n-Heptylcyclohexyl)-p'-n-pentyloxybiphenyl,
p-(4-n-Octylcyclohexyl)-p'-n-pentyloxybiphenyl,
p-(4-n-Nonylcyclohexyl)-p'-n-pentyloxybiphenyl,
p-(4-n-Decylcyclohexyl)-p'-n-pentyloxybiphenyl,
p-(4-n-Undecylcyclohexyl)-p'-n-pentyloxybiphenyl,
p-(4-n-Dodecylcyclohexyl)-p'-n-pentyloxybiphenyl,
p-[4-(2-Methylpropyl)-cyclohexyl]-p'-n-pentyloxybiphenyl,
p-[4-(2-Methylbutyl)-cyclohexyl]-p'-n-pentyloxybiphenyl,
p-[4-(3-Methylbutyl)-cyclohexyl]-p'-n-pentyloxybiphenyl,
p-[4-(2-Äthylhexyl)-cyclohexyl]-p'-n-pentyloxybiphenyl.

p-(4-Methylcyclohexyl)-p'-n-hexyloxybiphenyl,
p-(4-Äthylcyclohexyl)-p'-n-hexyloxybiphenyl,
p-(4-n-Propylcyclohexyl)-p'-n-hexyloxybiphenyl,
p-(4-n-Butylcyclohexyl)-p'-n-hexyloxybiphenyl,
p-(4-n-Pentylcyclohexyl)-p'-n-hexyloxybiphenyl,
p-(4-n-Hexylcyclohexyl)-p'-n-hexyloxybiphenyl,
p-(4-n-Heptylcyclohexyl)-p'-n-hexyloxybiphenyl,
p-(4-n-Octylcyclohexyl)-p'-n-hexyloxybiphenyl,
p-(4-n-Nonylcyclohexyl)-p'-n-hexyloxybiphenyl,
p-(4-n-Decylcyclohexyl)-p'-n-hexyloxybiphenyl,
p-[4-(2-Methylpropyl)-cyclohexyl]-p'-n-hexyloxybiphenyl,
p-[4-(2-Methylbutyl)-cyclohexyl]-p'-n-hexyloxybiphenyl,
p-[4-(3-Methylbutyl)-cyclohexyl]-p'-n-hexyloxybiphenyl,
p-[4-(2-Äthylhexyl)-cyclohexyl]-p'-n-hexyloxybiphenyl.

p-(4-Methylcyclohexyl)-p'-n-heptyloxybiphenyl,
p-(4-Äthylcyclohexyl)-p'-n-heptyloxybiphenyl,
p-(4-n-Propylcyclohexyl)-p'-n-heptyloxybiphenyl,
p-(4-n-Butylcyclohexyl)-p'-n-heptyloxybiphenyl,
p-(4-n-Pentylcyclohexyl)-p'-n-heptyloxybiphenyl,
p-(4-n-Hexylcyclohexyl)-p'-n-heptyloxybiphenyl,
p-(4-n-Heptylcyclohexyl)-p'-n-heptyloxybiphenyl,
p-(4-n-Octylcyclohexyl)-p'-n-heptyloxybiphenyl,
p-(4-n-Nonylcyclohexyl)-p'-n-heptyloxybiphenyl,
p-(4-n-Decylcyclohexyl)-p'-n-heptyloxybiphenyl,
p-[4-(2-Methylpropyl)-cyclohexyl]-p'-n-heptyloxybiphenyl,
p-[4-(2-Methylbutyl)-cyclohexyl]-p'-n-heptyloxybiphenyl,
p-[4-(3-Methylbutyl)-cyclohexyl]-p'-n-heptyloxybiphenyl,
p-[4-(2-Äthylhexyl)-cyclohexyl]-p'-n-heptyloxybiphenyl.

p-(4-Methylcyclohexyl)-p'-n-octyloxybiphenyl,
p-(4-Äthylcyclohexyl)-p'-n-octyloxybiphenyl,
p-(4-n-Propylcyclohexyl)-p'-n-octyloxybiphenyl,
p-(4-n-Butylcyclohexyl)-p'-n-octyloxybiphenyl,
p-(4-n-Pentylcyclohexyl)-p'-n-octyloxybiphenyl,
p-(4-n-Hexylcyclohexyl)-p'-n-octyloxybiphenyl,
p-(4-n-Heptylcyclohexyl)-p'-n-octyloxybiphenyl,
p-(4-n-Octylcyclohexyl)-p'-n-octyloxybiphenyl,
p-[4-(2-Methylpropyl)-cyclohexyl]-p'-n-octyloxybiphenyl,
p-[4-(2-Methylbutyl)-cyclohexyl]-p'-n-octyloxybiphenyl,
p-[4-(3-Methylbutyl)-cyclohexyl]-p'-n-octyloxybiphenyl,
p-[4-(2-Äthylhexyl)-cyclohexyl]-p'-n-octyloxybiphenyl.

p-(4-Methylcyclohexyl)-p'-n-nonyloxybiphenyl,
p-(4-Äthylcyclohexyl)-p'-n-nonyloxybiphenyl,
p-(4-n-Propylcyclohexyl)-p'-n-nonyloxybiphenyl,
p-(4-n-Butylcyclohexyl)-p'-n-nonyloxybiphenyl,
p-(4-n-Pentylcyclohexyl)-p'-n-nonyloxybiphenyl,
p-(4-n-Hexylcyclohexyl)-p'-n-nonyloxybiphenyl,
p-(4-n-Heptylcyclohexyl)-p'-n-nonyloxybiphenyl,
p-(4-n-Octylcyclohexyl)-p'-n-nonyloxybiphenyl,
p-[4-(2-Methylpropyl)-cyclohexyl]-p'-n-nonyloxybiphenyl,
p-[4-(2-Methylbutyl)-cyclohexyl]-p'-n-nonyloxybiphenyl,
p-[4-(3-Methylbutyl)-cyclohexyl]-p'-n-nonyloxybiphenyl.

p-(4-Methylcyclohexyl)-p'-n-decyloxybiphenyl,
p-(4-Äthylcyclohexyl)-p'-n-decyloxybiphenyl,
p-(4-n-Propylcyclohexyl)-p'-n-decyloxybiphenyl,
p-(4-n-Butylcyclohexyl)-p'-n-decyloxybiphenyl,
p-(4-n-Pentylcyclohexyl)-p'-n-decyloxybiphenyl,
p-(4-n-Hexylcyclohexyl)-p'-n-decyloxybiphenyl,
p-(4-n-Heptylcyclohexyl)-p'-n-decyloxybiphenyl,
p-(4-n-Octylcyclohexyl)-p'-n-decyloxybiphenyl,
p-[4-(2-Methylpropyl)-cyclohexyl]-p'-n-decyloxybiphenyl,
p-[4-(2-Methylbutyl)-cyclohexyl]-p'-n-decyloxybiphenyl,
p-[4-(3-Methylbutyl)-cyclohexyl]-p'-n-decyloxybiphenyl.

p-(4-Methylcyclohexyl)-p'-n-undecyloxybiphenyl,
p-(4-Äthylcyclohexyl)-p'-n-undecyloxybiphenyl,
p-(4-n-Propylcyclohexyl)-p'-n-undecyloxybiphenyl,
p-(4-n-Butylcyclohexyl)-p'-n-undecyloxybiphenyl,
p-(4-n-Pentylcyclohexyl)-p'-n-undecyloxybiphenyl,
p-(4-n-Hexylcyclohexyl)-p'-n-undecyloxybiphenyl,
p-(4-n-Heptylcyclohexyl)-p'-n-undecyloxybiphenyl,
p-[4-(2-Methylpropyl)-cyclohexyl]-p'-n-undecyloxybiphenyl,
p-[4-(2-Methylbutyl)-cyclohexyl]-p'-n-undecyloxybiphenyl,
p-[4-(3-Methylbutyl)-cyclohexyl]-p'-n-undecyloxybiphenyl.

p-(4-Methylcyclohexyl)-p'-n-dodecyloxybiphenyl,
p-(4-Äthylcyclohexyl)-p'-n-dodecyloxybiphenyl,
p-(4-n-Propylcyclohexyl)-p'-n-dodecyloxybiphenyl,
p-(4-n-Butylcyclohexyl)-p'-n-dodecyloxybiphenyl,
p-(4-n-Pentylcyclohexyl)-p'-n-dodecyloxybiphenyl,
p-(4-n-Hexylcyclohexyl)-p'-n-dodecyloxybiphenyl,
p-(4-n-Heptylcyclohexyl)-p'-n-dodecyloxybiphenyl,
p-[4-(2-Methylpropyl)-cyclohexyl]-p'-n-dodecyloxybiphenyl,
p-[4-(2-Methylbutyl)-cyclohexyl]-p'-n-dodecyloxybiphenyl.

p-(4-Methylcyclohexyl)-p'-(2-methylbutyloxy)-biphenyl,
p-(4-Äthylcyclohexyl)-p'-(2-methylbutyloxy)-biphenyl,
p-(4-n-Propylcyclohexyl)-p'-(2-methylbutyloxy)-biphenyl,
p-(4-n-Butylcyclohexyl)-p'-(2-methylbutyloxy)-biphenyl,
p-(4-n-Pentylcyclohexyl)-p'-(2-methylbutyloxy)-biphenyl,
p-(4-n-Hexylcyclohexyl)-p'-(2-methylbutyloxy)-biphenyl,
p-(4-n-Heptylcyclohexyl)-p'-(2-methylbutyloxy)-biphenyl,
p-(4-n-Octylcyclohexyl)-p'-(2-methylbutyloxy)-biphenyl,
p-(4-n-Nonylcyclohexyl)-p'-(2-methylbutyloxy)-biphenyl,
p-(4-n-Decylcyclohexyl)-p'-(2-methylbutyloxy)-biphenyl,
p-(4-n-Undecylcyclohexyl)-p'-(2-methylbutyloxy)-biphenyl,
p-(4-n-Dodecylcyclohexyl)-p'-(2-methylbutyloxy)-biphenyl.

p-(4-Methylcyclohexyl)-p'-(2-methylheptyloxy)-biphenyl,
p-(4-Äthylcyclohexyl)-p'-(2-methylheptyloxy)-biphenyl,
p-(4-n-Propylcyclohexyl)-p'-(2-methylheptyloxy)-biphenyl,
p-(4-n-Butylcyclohexyl)-p'-(2-methylheptyloxy)-biphenyl,
p-(4-n-Pentylcyclohexyl)-p'-(2-methylheptyloxy)-biphenyl,
p-(4-n-Hexylcyclohexyl)-p'-(2-methylheptyloxy)-biphenyl,
p-(4-n-Heptylcyclohexyl)-p'-(2-methylheptyloxy)-biphenyl,

p-(4-n-Octylcyclohexyl)-p'-(2-methylheptyloxy)-biphenyl,
p-(4-n-Nonylcyclohexyl)-p'-(2-methylheptyloxy)-biphenyl,
p-(4-n-Decylcyclohexyl)-p'-(2-methylheptyloxy)-biphenyl.

Beispiel 5

Eine Lösung von 32 g p-(4-n-Pentylcyclohexyl)-p'-hydroxybiphenyl in 200 ml Toluol wird mit 42 g Heptafluorbuttersäureanhydrid und 2 Tropfen konzentrierter Schwefelsäure 7 Stunden unter Rückfluß zum Sieden erhitzt. Das abgekühlte Reaktionsgemisch wird mit 150 ml wäßriger Natriumhydrogencarbonatlösung gewaschen, über Natriumsulfat getrocknet und eingedampft. Das zurückbleibende p-(4-n-Pentylcyclohexyl)-p'-heptafluorbutyryloxybiphenyl wird in einem Druckgefäß in Gegenwart von 25 g wasserfreier Flußsäure und 108 g Schwefeltetrafluorid 8 Stunden auf 170° erhitzt.
Anschließend werden die flüchtigen Anteile des Reaktionsgemisches durch Ausblasen mit Stickstoff entfernt, der Rückstand in einer Suspension von 50 g Natriumfluorid in 250 ml Tetrahydrofuran aufgenommen und die erhaltene Suspension nach 2stündigem Rühren filtriert. Das Filtrat wird eingedampft und das zurückbleibende p-(4-n-Pentylcyclohexyl)-p'-perfluorbutyloxybiphenyl aus Äthanol umkristallisiert.
Analog werden hergestellt:

p-(4-Methylcyclohexyl)-p'-perfluoräthoxybiphenyl,
p-(4-Äthylcyclohexyl)-p'-perfluoräthoxybiphenyl,
p-(4-n-Propylcyclohexyl)-p'-perfluoräthoxybiphenyl,
p-(4-n-Butylcyclohexyl)-p'-perfluoräthoxybiphenyl,
p-(4-n-Pentylcyclohexyl)-p'-perfluoräthoxybiphenyl,
p-(4-n-Hexylcyclohexyl)-p'-perfluoräthoxybiphenyl,
p-(4-n-Heptylcyclohexyl)-p'-perfluoräthoxybiphenyl,
p-(4-n-Octylcyclohexyl)-p'-perfluoräthoxybiphenyl,
p-(4-n-Nonylcyclohexyl)-p'-perfluoräthoxybiphenyl,
p-(4-n-Decylcyclohexyl)-p'-perfluoräthoxybiphenyl.

p-(4-Methylcyclohexyl)-p'-perfluorpropyloxybiphenyl,
p-(4-Äthylcyclohexyl)-p'-perfluorpropyloxybiphenyl,
p-(4-n-Propylcyclohexyl)-p'-perfluorpropyloxybiphenyl,
p-(4-n-Butylcyclohexyl)-p'-perfluorpropyloxybiphenyl,
p-(4-n-Pentylcyclohexyl)-p'-perfluorpropyloxybiphenyl,
p-(4-n-Hexylcyclohexyl)-p'-perfluorpropyloxybiphenyl,
p-(4-n-Heptylcyclohexyl)-p'-perfluorpropyloxybiphenyl,
p-(4-n-Octylcyclohexyl)-p'-perfluorpropyloxybiphenyl,
p-(4-n-Nonylcyclohexyl)-p'-perfluorpropyloxybiphenyl,
p-(4-n-Decylcyclohexyl)-p'-perfluorpropyloxybiphenyl.

p-(4-Methylcyclohexyl)-p'-perfluorbutyloxybiphenyl,
p-(4-Äthylcyclohexyl)-p'-perfluorbutyloxybiphenyl,
p-(4-n-Propylcyclohexyl)-p'-perfluorbutyloxybiphenyl,
p-(4-n-Butylcyclohexyl)-p'-perfluorbutyloxybiphenyl,
p-(4-n-Hexylcyclohexyl)-p'-perfluorbutyloxybiphenyl,
p-(4-n-Heptylcyclohexyl)-p'-perfluorbutyloxybiphenyl,
p-(4-n-Octylcyclohexyl)-p'-perfluorbutyloxybiphenyl,
p-(4-n-Nonylcyclohexyl)-p'-perfluorbutyloxybiphenyl,
p-(4-n-Decylcyclohexyl)-p'-perfluorbutyloxybiphenyl.

p-(4-Methylcyclohexyl)-p'-perfluorpentyloxybiphenyl,
p-(4-Äthylcyclohexyl)-p'-perfluorpentyloxybiphenyl,
p-(4-n-Propylcyclohexyl)-p'-perfluorpentyloxybiphenyl,
p-(4-n-Butylcyclohexyl)-p'-perfluorpentyloxybiphenyl,
p-(4-n-Pentylcyclohexyl)-p'-perfluorpentyloxybiphenyl,
p-(4-n-Hexylcyclohexyl)-p'-perfluorpentyloxybiphenyl,
p-(4-n-Heptylcyclohexyl)-p'-perfluorpentyloxybiphenyl,
p-(4-n-Octylcyclohexyl)-p'-perfluorpentyloxybiphenyl.

Beispiel 6

Analog Beispiel 1(a) wird aus 4-(4-n-Pentylcyclohexyl)-biphenyl und Trifluoracetylchlorid in Gegenwart von Aluminiumchlorid p-(4-n-Pentylcyclohexyl)-p'-trifluoracetylbiphenyl hergestellt. 40 g dieser Verbindung werden in einem Druckgefäß 8 Stunden mit 50 g Schwefeltetrafluorid auf 100° erhitzt. Nach dem Abkühlen wird das überschüssige Schwefeltetrafluorid und flüchtige Reaktionsprodukte durch Einblasen von Stickstoff entfernt und das zurückbleibende p-(4-n-Pentylcyclohexyl)-p'-perfluoräthylbiphenyl aus Äthanol umkristallisiert.

Analog werden hergestellt:

p-(4-Methylcyclohexyl)-p'-trifluormethylbiphenyl,
p-(4-Äthylcyclohexyl)-p'-trifluormethylbiphenyl,
p-(4-n-Propylcyclohexyl)-p'-trifluormethylbiphenyl,
p-(4-n-Butylcyclohexyl)-p'-trifluormethylbiphenyl,
p-(4-n-Pentylcyclohexyl)-p'-trifluormethylbiphenyl,
p-(4-n-Hexylcyclohexyl)-p'-trifluormethylbiphenyl,
p-(4-n-Heptylcyclohexyl)-p'-trifluormethylbiphenyl,
p-(4-n-Octylcyclohexyl)-p'-trifluormethylbiphenyl,
p-(4-n-Nonylcyclohexyl)-p'-trifluormethylbiphenyl,
p-(4-n-Decylcyclohexyl)-p'-trifluormethylbiphenyl.

p-(4-Methylcyclohexyl)-p'-perfluoräthylbiphenyl,
p-(4-Äthylcyclohexyl)-p'-perfluoräthylbiphenyl,
p-(4-n-Propylcyclohexyl)-p'-perfluoräthylbiphenyl,
p-(4-n-Butylcyclohexyl)-p'-perfluoräthylbiphenyl,
p-(4-n-Hexylcyclohexyl)-p'-perfluoräthylbiphenyl,
p-(4-n-Heptylcyclohexyl)-p'-perfluoräthylbiphenyl,
p-(4-n-Octylcyclohexyl)-p'-perfluoräthylbiphenyl,
p-(4-n-Nonylcyclohexyl)-p'-perfluoräthylbiphenyl,
p-(4-n-Decylcyclohexyl)-p'-perfluoräthylbiphenyl,
p-[4-(2-Methylpropyl)-cyclohexyl]-p'-perfluoräthylbiphenyl,
p-[4-(2-Methylbutyl)-cyclohexyl]-p'-perfluoräthylbiphenyl,
p-[4-(3-Methylbutyl)-cyclohexyl]-p'-perfluoräthylbiphenyl,
p-[4-(2-Äthylhexyl)-cyclohexyl]-p'-perfluoräthylbiphenyl.

p-(4-Methylcyclohexyl)-p'-perfluorpropylbiphenyl,
p-(4-Äthylcyclohexyl)-p'-perfluorpropylbiphenyl,
p-(4-n-Propylcyclohexyl)-p'-perfluorpropylbiphenyl,
p-(4-n-Butylcyclohexyl)-p'-perfluorpropylbiphenyl,
p-(4-n-Pentylcyclohexyl)-p'-perfluorpropylbiphenyl,
p-(4-n-Hexylcyclohexyl)-p'-perfluorpropylbiphenyl,
p-(4-n-Heptylcyclohexyl)-p'-perfluorpropylbiphenyl,
p-(4-n-Octylcyclohexyl)-p'-perfluorpropylbiphenyl,
p-(4-n-Nonylcyclohexyl)-p'-perfluorpropylbiphenyl,
p-(4-n-Decylcyclohexyl)-p'-perfluorpropylbiphenyl.

p-(4-Methylcyclohexyl)-p'-perfluorbutylbiphenyl,
p-(4-Äthylcyclohexyl)-p'-perfluorbutylbiphenyl,
p-(4-n-Propylcyclohexyl)-p'-perfluorbutylbiphenyl,
p-(4-n-Butylcyclohexyl)-p'-perfluorbutylbiphenyl,
p-(4-n-Pentylcyclohexyl)-p'-perfluorbutylbiphenyl,
p-(4-n-Hexylcyclohexyl)-p'-perfluorbutylbiphenyl,
p-(4-n-Heptylcyclohexyl)-p'-perfluorbutylbiphenyl,
p-(4-n-Octylcyclohexyl)-p'-perfluorbutylbiphenyl.

p-(4-Methylcyclohexyl)-p'-perfluorpentylbiphenyl,
p-(4-Äthylcyclohexyl)-p'-perfluorpentylbiphenyl,
p-(4-n-Propylcyclohexyl)-p'-perfluorpentylbiphenyl,
p-(4-n-Butylcyclohexyl)-p'-perfluorpentylbiphenyl,
p-(4-n-Pentylcyclohexyl)-p'-perfluorpentylbiphenyl,
p-(4-n-Hexylcyclohexyl)-p'-perfluorpentylbiphenyl,
p-(4-n-Heptylcyclohexyl)-p'-perfluorpentylbiphenyl,
p-(4-n-Octylcyclohexyl)-p'-perfluorpentylbiphenyl.

p-(4-Methylcyclohexyl)-p'-perfluorisobutylbiphenyl,
p-(4-Äthylcyclohexyl)-p'-perfluorisobutylbiphenyl,
p-(4-n-Propylcyclohexyl)-p'-perfluorisobutylbiphenyl,
p-(4-n-Butylcyclohexyl)-p'-perfluorisobutylbiphenyl,
p-(4-n-Pentylcyclohexyl)-p'-perfluorisobutylbiphenyl,
p-(4-n-Hexylcyclohexyl)-p'-perfluorisobutylbiphenyl,
p-(4-n-Heptylcyclohexyl)-p'-perfluorisobutylbiphenyl,
p-(4-n-Octylcyclohexyl)-p'-perfluorisobutylbiphenyl.

Die folgenden Beispiele betreffen erfindungsgemäße Dielektrika

Beispiel 7

Das Dielektrikum aus 67% 4-n-Butyl-4'-methoxyazoxybenzol und 33% 4-Äthyl-4'-methoxyazoxybenzol hat eine nematische Phase im Temperaturbereich von −5° bis +73° und eine Viskosität von $31.10^{-3}$ Pa · s bei 20°.
Ein Dielektrikum aus 95% dieser Zweikomponentenmischung und 5% p-(4-n-Pentylcyclohexyl)-p'-äthoxybiphenyl hat eine nematische Phase im Temperaturbereich von −10° bis +79° und eine Viskosität von $28.10^{-3}$ Pa · s bei 20°.

Beispiel 8

Das Dielektrikum aus
67% Anissäure-4-n-pentylphenylester und
33% 4-n-Hexyloxybenzoesäure-4'-n-pentylphenylester hat eine nematische Phase im Temperaturbereich von +15° bis +48° und eine Viskosität von $58.10^{-3}$ Pa · s bei 20°.
Ein Dielektrikum aus 80% dieser Zweikomponentenmischung und 20% p-(4-n-Pentylcyclohexyl)-p'-äthylbiphenyl hat eine nematische Phase im Temperaturbereich von 0° bis +71° und eine Viskosität von $49.10^{-3}$ Pa · s bei 20°.

Beispiel 9

Ein Dielektrikum aus 77% der Zweikomponentenmischung gemäß Beispiel 7 und 23% p-(4-n-Pentylcyclohexyl)-p'-äthylbiphenyl hat eine nematische Phase im Temperaturbereich von −15° bis +95° und eine Viskosität von $30.10^{-3}$ Pa · s bei 20°.

Beispiel 10

Ein Dielektrikum aus 80% der Zweikomponentenmischung gemäß Beispiel 8 und 20% p-(4-n-Hexylcyclohexyl)-p'-äthylbiphenyl hat eine nematische Phase im Temperaturbereich von 6° bis 68° und eine Viskosität von $52.10^{-3}$ Pa · s bei 20°.

Beispiel 11

Das Dielektrikum aus 29% 4-(4-n-Propylcyclohexyl)-benzonitril, 41% 4-(4-n-Pentylcyclohexyl)-benzonitril und 30% 4-(4-n-Heptylcyclohexyl)-benzonitril hat eine nematische Phase im Temperaturbereich von −3° bis +52° und eine Viskosität von 24 cSt bei 20°.
Ein Dielektrikum aus 74% dieses ternären Gemisches und 26% p-(4-n-Pentylcyclohexyl)-p'-äthylbiphenyl hat eine nematische Phase im Temperaturbereich von −10° bis +74° und eine Viskosität von 23 cSt bei 20°.

Beispiel 12

Die flüssigkristalline Verbindung p-n-Pentyl-p'-cyanobiphenyl hat eine nematische Phase im Temperaturbereich von 22,5° bis 35° und eine Viskosität von 29,5 cSt bei 20° (unterkühlter Zustand).
Ein Gemisch aus 90% p-n-Pentyl-p'-cyclobiphenyl und 10% p-(4-n-Pentylcyclohexyl)-p'-n-butylbiphenyl hat eine nematische Phase im Temperaturbereich von 18,5° bis 49,2° und eine Viskosität von 29 cSt bei 20°.

**Patentansprüche für die Vertragsstaaten CH, LI, DE, FR, GB, IT, NL**

1. Cyclohexylbiphenyle der Formel (I)

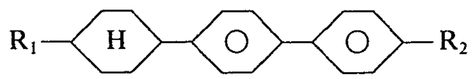 (I)

worin

$R_1$ eine Alkylgruppe mit 1 – 12 C-Atomen und
$R_2$ eine gegebenenfalls perfluorierte Alkyl-, Alkoxy- oder Alkanoyloxygruppe mit 1 – 12 C-Atomen

bedeutet, wobei jedoch höchstens einer der Substituenten $R_1$ und $R_2$ eine verzweigte Kohlenstoffkette enthält.

2. Verfahren zur Herstellung der Cyclohexylbiphenyle der Formel (I)

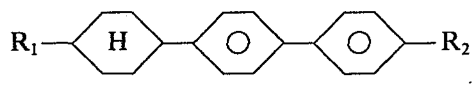 (I)

worin

$R_1$ eine Alkylgruppe mit 1 – 12 C-Atomen und
$R_2$ eine gegebenenfalls perfluorierte Alkyl-, Alkoxy- oder Alkanoyloxygruppe mit 1 – 12 C-Atomen

bedeutet, wobei jedoch höchstens einer der Substituenten $R_1$ und $R_2$ eine verzweigte Kohlenstoffkette enthält, dadurch gekennzeichnet, daß man

(a) zur Herstellung einer Verbindung der Formel (I), worin $R_2$ eine Alkylgruppe bedeutet, in einer Verbindung der Formel (II)

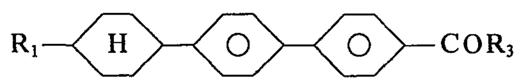 (II)

worin $R_3$ H oder Alkyl mit 1 – 11 C-Atomen bedeutet und $R_1$ die vorstehend angegebene Bedeutung besitzt, die Carbonylgruppe zur Methylengruppe reduziert, oder

(b) zur Herstellung einer Verbindung der Formel (I), worin $R_2$ eine Alkoxygruppe bedeutet, eine Verbindung der Formel (II) durch Behandlung mit einem Oxidationsmittel und gegebenenfalls anschließende Hydrolyse in ein Phenol der Formel (III)

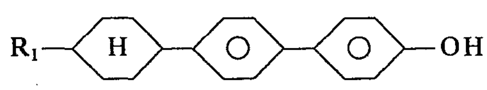 (III)

worin $R_1$ die vorstehend angegebene Bedeutung besitzt, überführt, und dieses mit einem O-alkylierenden Mittel umsetzt, oder

(c) zur Herstellung einer Verbindung der Formel (I), worin $R_2$ eine Perfluoralkylgruppe bedeutet, ein 4-(4-Alkylcyclohexyl)-biphenyl mit einem Perfluorcarbonsäurehalogenid in Gegenwart einer Lewis-Säure umsetzt und in dem so erhaltenen Fluoralkylketon die Carbonylgruppe durch Umsetzung mit Schwefeltetrafluorid in eine Difluormethylengruppe überführt, oder

(d) zur Herstellung einer Verbindung der Formel (I), worin $R_2$ eine Perfluoralkoxygruppe bedeutet, ein Phenol der Formel (III) durch Umsetzung mit einem Perfluorcarbonsäurederivat verestert und in dem erhaltenen Perfluorcarbonsäurephenylesterderivat die Estercarbonylgruppe durch Umsetzung mit Schwefeltetrafluorid in eine Difluormethylengruppe überführt, oder

(e) zur Herstellung einer Verbindung der Formel (I), worin $R_2$ eine gegebenenfalls perfluorierte Alkanoyloxygruppe bedeutet, ein Phenol der Formel (III) durch Umsetzung mit einer gegebenenfalls perfluorierten Alkancarbonsäure oder einem reaktiven Derivat einer solchen Säure in an sich üblicher Weise verestert.

3. Verwendung der Cyclohexylbiphenyle der Formel (I)

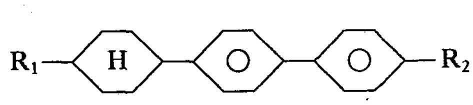 (I)

worin

$R_1$ eine Alkylgruppe mit 1 – 12 C-Atomen und
$R_2$ eine gegebenenfalls perfluorierte Alkyl-, Alkoxy- oder Alkanoyloxygruppe mit 1 – 12 C-Atomen

bedeutet, wobei jedoch höchstens einer der Substituenten $R_1$ und $R_2$ eine verzweigte Kohlenstoffkette enthält, als Komponenten flüssigkristalliner Dielektrika.

4. Flüssigkristallines Dielektrikum aus 2 oder mehreren Komponenten, dadurch gekennzeichnet, daß es mindestens ein Cyclohexylbiphenyl der Formel (I) enthält

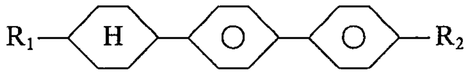 (I)

worin

$R_1$ eine Alkylgruppe mit 1 – 12 C-Atomen und
$R_2$ eine gegebenenfalls perfluorierte Alkyl-, Alkoxy- oder Alkanoyloxygruppe mit 1 – 12 C-Atomen

bedeutet, wobei jedoch höchstens einer der Substituenten $R_1$ und $R_2$ eine verzweigte Kohlenstoffkette enthält.

5. Elektrooptisches Anzeigeelement auf der Basis einer Flüssigkristallzelle, dadurch gekennzeichnet, daß es ein flüssigkristallines Dielektrikum nach Anspruch 4 enthält.

**Patentansprüche für den Vertragsstaat AT**

1. Verfahren zur Herstellung der Cyclohexylbiphenyle der Formel (I)

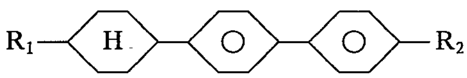 (I)

worin

$R_1$ eine Alkylgruppe mit 1 – 12 C-Atomen und
$R_2$ eine gegebenenfalls perfluorierte Alkyl-, Alkoxy- oder Alkanoyloxygruppe mit 1 – 12 C-Atomen

bedeutet, wobei höchstens einer der Substituenten $R_1$ und $R_2$ eine verzweigte Kohlenstoffkette enthält, dadurch gekennzeichnet, daß man

(a) zur Herstellung einer Verbindung der Formel (I), worin $R_2$ eine Alkylgruppe bedeutet, in einer Verbindung der Formel (II)

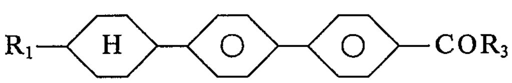 (II)

worin $R_3$ H oder Alkyl mit 1 – 11 C-Atomen bedeutet und $R_1$ die vorstehend angegebene Bedeutung besitzt, die Carbonylgruppe zur Methylengruppe reduziert, oder

(b) zur Herstellung einer Verbindung der Formel (I), worin $R_2$ eine Alkoxygruppe bedeutet, eine Verbindung der Formel (II) durch Behandlung mit einem Oxidationsmittel und gegebenenfalls anschließende Hydrolyse in ein Phenol der Formel (III)

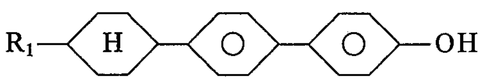 (III)

worin $R_1$ die vorstehend angegebene Bedeutung besitzt, überführt, und dieses mit einem O-alkylierenden Mittel umsetzt, oder

(c) zur Herstellung einer Verbindung der Formel (I), worin $R_2$ eine Perfluoralkylgruppe bedeutet, ein 4-(4-Alkylcyclohexyl)-biphenyl mit einem Perfluorcarbonsäurehalogenid in Gegenwart einer Lewis-Säure umsetzt und in dem so erhaltenen Fluoralkylketon die Carbonylgruppe durch Umsetzung mit Schwefeltetrafluorid in eine Difluormethylengruppe überführt, oder

(d) zur Herstellung einer Verbindung der Formel (I), worin $R_2$ eine Perfluoralkoxygruppe bedeutet, ein

Phenol der Formel (III) durch Umsetzung mit einem Perfluorcarbonsäurederivat verestert und in dem erhaltenen Perfluorcarbonsäurephenylesterderivat die Estercarbonylgruppe durch Umsetzung mit Schwefeltetrafluorid in eine Difluormethylengruppe überführt, oder

(e) zur Herstellung einer Verbindung der Formel (I), worin $R_2$ eine gegebenenfalls perfluorierte Alkanoyloxygruppe bedeutet, ein Phenol der Formel (III) durch Umsetzung mit einer gegebenenfalls perfluorierten Alkancarbonsäure oder einem reaktiven Derivat einer solchen Säure in an sich üblicher Weise verestert.

2. Verwendung der Cyclohexylbiphenyle der Formel (I)

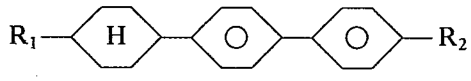
(I)

worin

$R_1$ eine Alkylgruppe mit 1–12 C-Atomen und
$R_2$ eine gegebenenfalls perfluorierte Alkyl-, Alkoxy- oder Alkanoyloxygruppe mit 1–12 C-Atomen

bedeutet, wobei jedoch höchstens einer der Substituenten $R_1$ und $R_2$ eine verzweigte Kohlenstoffkette enthält, als Komponenten flüssigkristalliner Dielektrika.

3. Flüssigkristallines Dielektrikum aus 2 oder mehreren Komponenten, dadurch gekennzeichnet, daß es mindestens ein Cyclohexylbiphenyl der Formel (I) enthält,

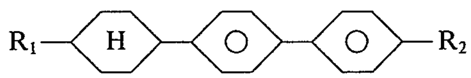
(I)

worin

$R_1$ eine Alkylgruppe mit 1–12 C-Atomen und
$R_2$ eine gegebenenfalls perfluorierte Alkyl-, Alkoxy- oder Alkanoyloxygruppe mit 1–12 C-Atomen

bedeutet, wobei jedoch höchstens einer der Substituenten $R_1$ und $R_2$ eine verzweigte Kohlenstoffkette enthält.

4. Elektrooptisches Anzeigeelement auf der Basis einer Flüssigkristallzelle, dadurch gekennzeichnet, daß es ein flüssigkristallines Dielektrikum nach Anspruch 3 enthält.

**Claims for the Contracting states: CH, LI, DE, FR, GB, IT, NL**

1. Cyclohexylbiphenyls of the formula (I)

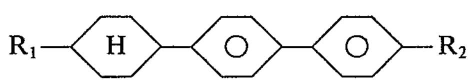
(I)

wherein $R_1$ is an alkyl group with 1–12 C atoms and $R_2$ is an optionally perfluorinated alkyl, alkoxy or alkanoyloxy group with 1–12 C atoms, wherein, however, at most one of the substituents $R_1$ and $R_2$ contains a branched carbon chain.

2. Process for the preparation of the cyclohexylbiphenyls of the formula (I)

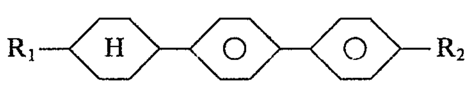
(I)

wherein $R_1$ is an alkyl group with 1–12 C atoms and $R_2$ is an optionally perfluorinated alkyl, alkoxy or alkanoyloxy group with 1–12 C atoms, wherein, however, at most one of the substituents $R_1$ and $R_2$ contains a branched carbon chain, characterised in that

(a) for the preparation of a compound of the formula (I) wherein $R_2$ denotes an alkyl group, the carbonyl group in a compound of the formula (II)

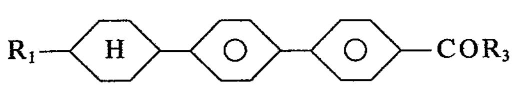
(II)

wherein $R_3$ is H or alkyl with 1 – 11 C atoms and $R_1$ has the abovementioned meaning, is reduced to the methylene group, or

(b) to prepare a compound of the formula (I) wherein $R_2$ is an alkoxy group, a compound of the formula (II) is converted into a phenol of the formula (III)

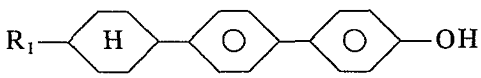

(III)

wherein $R_1$ has the abovementioned meaning, by treatment with an oxidising agent and, if appropriate, subsequent hydrolysis, and this phenol ist reacted with an O-alkylating agent, or

(c) to prepare a compound of the formula (I) wherein $R_2$ is a perfluoroalkyl group, a 4-(4-alkylcyclohexyl)-bi-phenyl is reacted with a perfluorocarboxylic acid halide in the presence of a Lewis acid ans the carbonyl group in the fluoroalkyl ketone thus obtained is converted into a difluoromethylene group by reaction with sulfur tetrafluoride, or

(d) to prepare a compound of the formula (I) wherein $R_2$ is a perfluoroalkoxy group, a phenol of the formula (III) is esterified by reaction with a perfluorocarboxylic acid derivative and the ester-carbonyl group in the resulting perfluorocarboxylic acid phenyl ester derivative is converted into a difluoromethylene group by reaction with sulfur tetrafluoride, or

(e) to prepare a compound of the formula (I) wherein $R_2$ is an optionally perfluorinated alkanoyloxy group, a phenol of the formula (III) is esterified in a manner which is in itself customary, by reaction with an optionally perfluorinated alkanecarboxylic acid or a reactive derivative of such an acid.

3. Use of the cyclohexylbiphenyls of the formula (I)

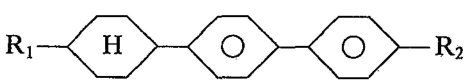

(I)

wherein $R_1$ is an alkyl group with 1 – 12 C atoms and $R_2$ is an optionally perfluorinated alkyl, alkoxy or alkanoyloxy group with 1 – 12 C atoms, wherein, however, at most one of the substituents $R_1$ and $R_2$ contains a branched carbon chain, as components of liquid crystal dielectrics.

4. Liquid crystal dielectric, chracterised in that it contains at least one cyclohexylbiphenyl of the formula (I)

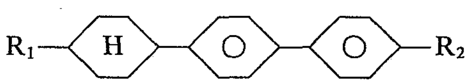

(I)

wherein $R_1$ is an alkyl group with 1 – 12 C atoms and $R_2$ is an optionally perfluorinated alkyl, alkoxy or alkanoyloxy group with 1 – 12 C atoms, wherein, however, at most one of the substituents $R_1$ and $R_2$ contains a branched carbon chain.

5. Electrooptical display element based on a liquid crystal cell, characterised in that it contains a liquid crystal dielectric according to claim 4.

**Claims for the Contracting state AT**

1. Process for the preparation of the cyclohexylbiphenyls of the formula (I)

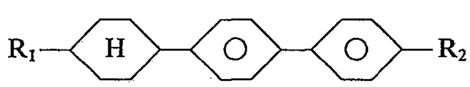

(I)

wherein $R_1$ is an alkyl group with 1 – 12 C atoms and $R_2$ is an optionally perfluorinated alkyl, alkoxy or alkanoyloxy group with 1 – 12 C atoms, wherein, however, at most one of the substituents $R_1$ and $R_2$ contains a branched carbon chain, characterised in that

(a) for the preparation of a compound of the formula (I) wherein $R_2$ denotes an alkyl group, the carbonyl group in a compound of the formula (II)

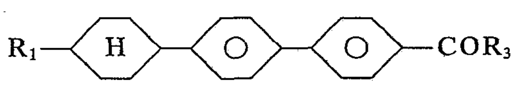

(II)

wherein $R_3$ is H or alkyl with 1–11 C atoms and $R_1$ has the abovementioned meaning, is reduced to the methylene group, or

(b) to prepare a compound of the formula (I) wherein $R_2$ is an alkoxy group, a compound of the formula (II) is converted into a phenol of the formula (III)

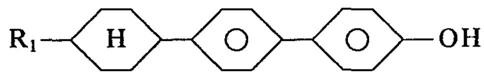

(III)

wherein $R_1$ has the abovementioned meaning, by treatment with an oxidising agent and, if appropriate, subsequent hydrolysis, and this phenol is reacted with an O-alkylating agent, or

(c) to prepare a compound of the formula (I) wherein $R_2$ is a perfluoroalkyl group, a 4-(4-alkylcyclohexyl)-biphenyl is reacted with a perfluorocarboxylic acid halide in the presence of a Lewis acid and the carbonyl group in the fluoroalkyl ketone thus obtained is converted into a difluoromethylene group by reaction with sulfur tetrafluoride, or

(d) to prepare a compound of the formula (I) wherein $R_2$ is a perfluoroalkoxy group, a phenol of the formula (III) is esterified by reaction with a perfluorocarboxylic acid derivative and the ester-carbonyl group in the resulting perfluorocarboxylic acid phenyl ester derivative is converted into a difluoromethylene group by reaction with sulfur tetrafluoride, or

(e) to prepare a compound of the formula (I) wherein $R_2$ is an optionally perfluorinated alkanoyloxy group, a phenol of the formula (III) is esterified in a manner which is in itself customary, by reaction with an optionally perfluorinated alkanecarboxylic acid or a reactive derivative of such an acid.

2. Use of the cyclohexylbiphenyls of the formula (I)

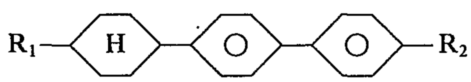

(I)

wherein $R_1$ is an alkyl group with 1–12 C atoms and $R_2$ is an optionally perfluorinated alkyl, alkoxy or alkanoyloxy group with 1–12 C atoms, wherein, however, at most one of the substituents $R_1$ and $R_2$ contains a branched carbon chain, as components of liquid crystal dielectrics.

3. Liquid crystal dielectric, characterised in that it contains at least one cyclohexylbiphenyl of the formula (I)

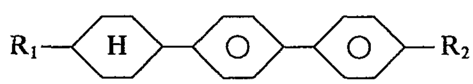

(I)

wherein $R_1$ is an alkyl group with 1–12 C atoms and $R_2$ is an optionally perfluorinated alkyl, alkoxy or alkanoyloxy group with 1–12 C atoms, wherein, however, at most one of the substituents $R_1$ and $R_2$ contains a branched carbon chain.

4. Electrooptical display element based on a liquid crystal cell, characterised in that it contains a liquid crystal dielectric according to claim 4.

**Revendications pour les Etats contractants: CH, LI, DE, FR, GB, IT, NL**

1. Cyclohexylbiphényles de formule (I)

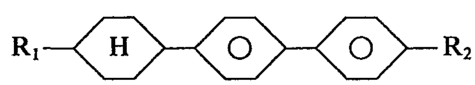

(I)

où $R_1$ représente un groupe alcoyle en $C_1$ à $C_{12}$ et $R_2$ un groupe alcoyle, alcoxy ou alcanoyloxy en $C_1$ à $C_{12}$ éventuellement perfluoré, où cependant au plus l'un des substituants $R_1$ et $R_2$ contient une chaîne hydrocarbonée ramifiée.

2. Procédé de préparation des cyclohexylbiphényles de formule (I)

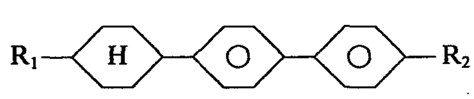

(I)

où $R_1$ représente un groupe alcoyle en $C_1$ à $C_{12}$ et $R_2$ un groupe alcoyle, alcoxy ou alcanoyloxy en $C_1$ à $C_{12}$

éventuellement perfluoré, où cependant au plus l'un des substituants $R_1$ et $R_2$ contient une chaîne hydrocarbonée ramifiée, caractérisé en ce que

(a) Pour préparer un composé de formule (I) où $R_2$ représente un groupe alcoyle, on réduit le groupe carbonyl en groupe méthylène dans un composé de formule (II)

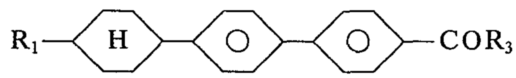

(II)

où $R_3$ représente H ou un alcoyle en $C_1$ à $C_{11}$ et où $R_1$ possède la signification donnée ci-dessus, ou

(b) Pour préparer un composé de formule (I) où $R_2$ représente un groupe alcoxy, on transforme un composé de formule (II) par traitement avec un agent oxydant suivi éventuellement par une hydrolyse en un phénol de formule (III)

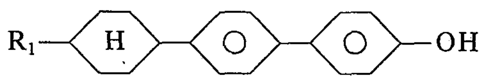

(III)

où $R_1$ possède la signification donnée ci-dessus, et en ce qu'on fait réagir celui-ci avec un agent O-alcoylant, ou

(c) Pour préparer un composé de formule (I) où $R_2$ représente un groupe perfluoroalcoyle, on fait réagir un 4-(4-alcoylcyclohexyl)-biphényle avec un halogénure d'acide perfluorocarboxylique en présence d'un acide de Lewis et on transforme dans la fluoroalcoylcétone ainsi obtenue le groupe carbonyle par réaction avec du tétrafluorure de soufre en un groupe difluorométhylène, ou

(d) Pour préparer un composé de formule (I) où $R_2$ représente un groupe perfluoroalcoxy, on estérifie un phénol de formule (III) par réaction avec un dérivé d'acide perfluorocarboxylique et dans le dérivé de phénylester d'acide perfluorocarboxylique obtenu, on transforme le groupe estercarbonyle par réaction avec du tétrafluorure de soufre en un groupe difluorométhylène, ou

(e) Pour préparer un composé de formule (I) où $R_2$ représente un groupe alcanoyloxy éventuellement perfluoré, on estérifie de manière habituelle un phénol de formule (III) par réaction avec un acide alcanecarboxylique éventuellement prefluoré ou un dérivé réactif d'un tel acide.

3. Application des cyclohexylbiphényles de formule (I)

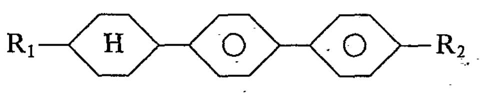

(I)

où

$R_1$ représente un groupe alcoyle en $C_1$ à $C_{12}$ et
$R_2$ un groupe alcoyle, alcoxy ou alcanoyloxy en $C_1$ à $C_{12}$ éventuellement perfluoré,

où cependant au plus l'un des substituants $R_1$ et $R_2$ contient une chaîne carbonée ramifiée, comme composants de diélectriques à cristaux liquides.

4. Diélectrique à cristaux liquides constitué de deux ou plusieurs composants, caractérisé en ce qu'il contient au moins un cyclohexylbiphényle de formule (I)

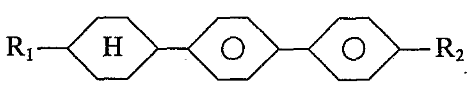

(I)

où

$R_1$ représente un groupe alcoyle en $C_1$ à $C_{12}$ et
$R_2$ un groupe alcoyle, alcoxy ou alcanoyloxy en $C_1$ à $C_{12}$ éventuellement perfluoré,

où cependant au plus l'un des substituants $R_1$ et $R_2$ contient une chaîne carbonée ramifiée.

5. Elément indicateur électro-optique à base d'une cellule à cristaux liquides, caractérisé en ce qu'il contient un diélectrique à cristaux liquides selon la revendication 4.

**Revendications pour l'Etat contractant: AT**

1. Procédé de préparation des cyclohexylbiphényles de formule (I)

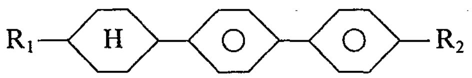

(I)

où $R_1$ représente un groupe alcoyle en $C_1$ à $C_{12}$ et $R_2$ un groupe alcoyle, alcoxy ou alcanoyloxy en $C_1$ à $C_{12}$ éventuellement perfluoré, où cependant au plus l'un des substituants $R_1$ et $R_2$ contient une chaîne hydrocarbonée ramifiée, caractérisé en ce que

(a) Pour préparer un composé de formule (I) où $R_2$ représente un groupe alcoyle, on réduit le groupe carbonyle en groupe méthylène dans un composé de formule (II)

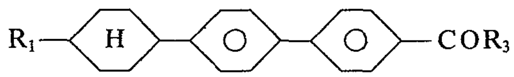

(II)

où $R_3$ représente H ou un alcoyle en $C_1$ à $C_{11}$ et où $R_1$ possède la signification donnée ci-dessus, ou

(b) Pour préparer un composé de formule (I) où $R_2$ représente un groupe alcoxy, on transforme un composé de formule (II) par traitement avec un agent oxydant suivi éventuellement par une hydrolyse en un phénol de formule (III)

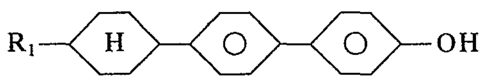

(III)

où $R_1$ possède la signification donnée ci-dessus, et en ce qu'on fait réagir celui-ci avec un agent O-alcoylant, ou

(c) Pour préparer un composé de formule (I) où $R_2$ représente un groupe perfluoroalcoyle, on fait réagir un 4-(4-alcoylcyclohexyl)-biphényle avec un halogénure d'acide perfluorocarboxylique en présence d'un acide de Lewis et on transforme dans la fluoroalcoylcétone ainsi obtenue le groupe carbonyle par réaction avec du tétrafluorure de soufre en un groupe difluorométhylène, ou

(d) Pour préparer un composé de formule (I) où $R_2$ représente un groupe perfluoroalcoxy, on estérifie un phénol de formule (III) par réaction avec un dérivé d'acide perfluorocarboxylique et dans le dérivé de phénylester d'acide perfluorocarboxylique obtenu, on transforme le groupe estercarbonyle par réaction avec du tétrafluorure de soufre en un groupe difluorométhylène, ou

(e) Pour préparer un composé de formule (I) où $R_2$ représente un groupe alcanoyloxy éventuellement perfluoré, on estérifie de manière habituelle un phénol de formule (III) par réaction avec un acide alcanecarboxylique éventuellement perfluoré ou un dérivé réactif d'un tel acide.

2. Application des cyclohexylbiphényles de formule (I)

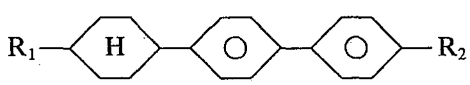

(I)

où

$R_1$ représente un groupe alcoyle en $C_1$ à $C_{12}$ et
$R_2$ un groupe alcoyle, alcoxy ou alcanoyloxy en $C_1$ à $C_{12}$ éventuellement perfluoré,

où cependant au plus l'un des substituants $R_1$ et $R_2$ contient une chaîne carbonée ramifiée, comme composants de diélectriques à cristaux liquides.

3. Diélectrique à cristaux liquides constitué de deux ou plusieurs composants, caractérisé en ce qu'il contient au moins un cyclohexylbiphényle de formule (I)

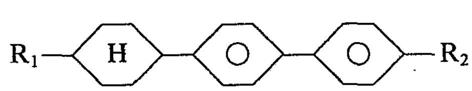

(I)

où

$R_1$ représente un groupe alcoyle en $C_1$ à $C_{12}$ et
$R_2$ un groupe alcoyle, alcoxy ou alcanoyloxy en $C_1$ à $C_{12}$ éventuellement perfluoré,

où cependant au plus l'un des substituants $R_1$ et $R_2$ contient une chaîne carbonée ramifiée.

4. Elément indicateur électro-optique à base d'une cellule à cristaux liquides, caractérisé en ce qu'il contient un diélectrique à cristaux liquides selon la revendication 3.